# EUROPEAN PATENT APPLICATION

(11) **EP 4 446 342 A1**
(43) Date of publication of application: **16.10.2024**
(21) Application number: 22903631.4
(22) Date of filing: 12.12.2022
(51) Int. Cl.: C07K 16/28, C07K 16/30, A61K 39/395, A61P 35/00

(54) **MULTI-SPECIFIC T CELL ENGAGERS COMPRISING LRRC15 ANTIGEN-BINDING DOMAIN**

(30) Priority: 10.12.2021 CN 202111509659
(71) Applicant: Suzhou Zelgen Biopharmaceutical Co., Ltd., Jiangsu 215300 (CN); Shanghai Zelgen Pharma.Tech Co., Ltd., Shanghai 201203 (CN)
(72) Inventor: LIN, Feng, Shanghai 201203 (CN); ZHANG, Bin, Shanghai 201203 (CN); DAI, Tongcheng, Shanghai 201203 (CN); SHENG, Jackie, Shanghai 201203 (CN); LV, Binhua, Shanghai 201203 (CN); SHENG, Zelin, Shanghai 201203 (CN)
(74) Representative: dompatent von Kreisler Selting Werner - Partnerschaft von Patent- und Rechtsanwälten mbB
(86) International application number: PCT/CN2022/138466
(87) International publication number: WO 2023/104214

(57) **Abstract**

The present invention relates to multi-specific T cell engagers and the use thereof. Specifically, the present invention provides the multi-specific T cell engager, comprising a first targeting domain targeting leucine-rich repeat containing protein 15 (LRRC15) molecules highly expressed on the surface of a tumor cell, and further comprisings a second targeting domain and/or a third targeting domain bound to the CD molecules of T cell surface. The LRRC15 related multi-specific T cell engagers of the present invention can effectively activate T cells and reduce the tumor stroma impact, and can be used as an effective therapeutic agent for treatment of tumors, especially tumors with a high expression level of LRRC15.

## Description

### TECHNICAL FIELD

The present invention relates to the technical field of cancer therapy, in particular, to multi-specific T cell engagers comprising anti-LRCC15 antibody or immunoreactive fragments thereof for cancer treatment.

### BACKGROUND

Cancer is generally defined as a group of diseases involving abnormal cell growth with the potential to invade or spread to other parts of the body. Conventional cancer treatments are aimed at eliminating cancer tissue and preventing its spread. Such treatment options include surgery, chemotherapy, radiotherapy, hormone therapy, targeted therapy, and palliative care. Treatment is usually based on the type, location, and stage of the cancer, as well as the patient's health and preferences. However, these therapies have limitations, as they may be ineffective, especially when the cancer has already metastasized. In addition, chemotherapy and radiotherapy have a range of side effects associated with their cytotoxicity.

Currently, promising areas of cancer treatment include the use of antibodies for targeted therapy. Another promising area is the development of therapies that utilize the immune system to attack and kill tumor cells.

Leucine-rich repeat containing 15 (LRRC15) is a member of the leucine-rich repeat (LRR) superfamily. LRRC15 is highly expressed on tumor cells, including breast cancer, brain tumors, pancreatic cancer, lung cancer, melanoma, highly invasive sarcomas, and breast cancer bone metastasis, while its expression level in normal tissues is relatively low. In addition, it is also highly expressed on stromal fibroblasts in many solid tumors (e.g., breast, head and neck, lung, pancreas). Therefore, LRRC15 is a candidate for targeted therapies such as antibody drug conjugates, T cell engagers, and CAR-T cells.

CD3, CD28, and CD137 are receptors that are present on T cells. T cells can be activated by antigen-presenting cells through CD3, CD28, and CD137, which utilize MHC Class I/II, CD80 and CD86, and 4-1BBL, respectively. CD3 is part of the T cell receptor (TCR) and is the signaling component of the receptor. CD3 has 3 subunits, epsilon, delta, and gamma. Epsilon is associated with both delta and zeta, which together are responsible for signal transduction. CD3 signaling is considered to be signal 1 required for T cell activation. The co-receptors CD28 and CD137 are considered to be signal 2. Both signal 1 and signal 2 are necessary for full activation, proliferation, and survival of T cells.

CD40 ligand (CD40L) is mainly expressed by activated T cells. CD40L binds to CD40 on antigen-presenting cells (APCs), thereby inducing APC activation. In turn, APCs induce cytotoxic T lymphocytes (CTLs). CD40 and CD40L are costimulatory molecules that link innate and adaptive immunity.

Despite the increasing number of therapeutic monoclonal antibodies approved for the treatment of various cancers, resistance to these antibodies is frequently observed, given that cancer growth and metastasis development have many different molecular pathways. Although the immune system is the main mechanism for preventing cancer, cancer cells evade immune surveillance. Therefore, there is an urgent need in the field for improved therapeutic combinations of antagonists or antibodies and methods of using these agents to treat cancer.

### SUMMARY OF THE INVENTION

The present invention provides bispecific/trispecific T cell engagers comprising an LRRC15 antigen binding domain. The bispecific/trispecific T cell engager molecule can bind to LRRC15 and activate CD (cluster of differentiation) molecules (e.g., CD3, CD28, CD137, and CD40). Methods for treating diseases such as cancer using the antibodies and antibody conjugates of the present invention, pharmaceutical compositions and products thereof are also provided.

In the first aspect of the present invention, it provides a multi-specific T cell engager, which comprises:
A first targeting domain comprising one or more LRRC15 antigen binding domains;
A second targeting domain, wherein the second targeting domain binds to a target protein selected from the group consisting of: CD3, CD28, CD40, and CD137; and/or
optionally, a third targeting domain, wherein the third targeting domain binds to a target protein selected from the group consisting of: CD3, CD28, CD40, and CD137.

In another preferred embodiment, the targeting domain is in the form of a single domain antibody (sdAb), a fragment variable (Fv) heterodimer, a single chain Fv (scFv), a Fab fragment, a TriFab, or a combination thereof.

In another preferred embodiment, the LRRC15 antigen binding domain comprises the following three heavy chain variable region CDRs:
HCDR1 having an amino acid sequence as shown in SEQ ID NO: 32, 35, 38, or 41;
HCDR2 having an amino acid sequence as shown in SEQ ID NO: 33, 36, 39, or 42; and
HCDR3 having an amino acid sequence as shown in SEQ ID NO: 34, 37, 40, or 43;

And, the following three light chain variable region CDRs:
LCDR1 having an amino acid sequence as shown in SEQ ID NO: 44 or 49;
LCDR2 having an amino acid sequence as shown in SEQ ID NO: 45, 47, or 50; and
LCDR3 having an amino acid sequence as shown in SEQ ID NO: 46, 48, 51, or 52.

In another preferred embodiment, the LRRC15 antigen binding domain comprises three heavy chain variable region CDRs (HCDRs), and three light chain variable region CDRs (LCDRs) selected from the group consisting of:

| Antibody Name | SEQ ID NO of HCDR1 | SEQ ID NO of HCDR2 | SEQ ID NO of HCDR3 | SEQ ID NO of LCDR1 | SEQ ID NO of LCDR2 | SEQ ID NO of LCDR3 |
|---|---|---|---|---|---|---|
| L15-06/ L15-06C | 32 | 33 | 34 | 44 | 45 | 46 |
| L15-08 | 35 | 36 | 37 | 44 | 47 | 48 |
| L15-03 | 38 | 39 | 40 | 49 | 50 | 51 |
| L15-07 | 41 | 42 | 43 | 49 | 50 | 52 |

In another preferred embodiment, the LRRC15 antigen binding domain comprises the following three heavy chain variable region CDRs:
HCDR1 having an amino acid sequence as shown in SEQ ID NO: 32;
HCDR2 having an amino acid sequence as shown in SEQ ID NO: 33; and
HCDR3 having an amino acid sequence as shown in SEQ ID NO: 34;
And, the following three light chain variable region CDRs:
   LCDR1 having an amino acid sequence as shown in SEQ ID NO: 44;
   LCDR2 having an amino acid sequence as shown in SEQ ID NO: 45; and
   LCDR3 having an amino acid sequence as shown in SEQ ID NO: 46.

In another preferred embodiment, the LRRC15 antigen binding domain comprises the following three heavy chain variable region CDRs:
HCDR1 having an amino acid sequence as shown in SEQ ID NO: 35;
HCDR2 having an amino acid sequence as shown in SEQ ID NO: 36; and
HCDR3 having an amino acid sequence as shown in SEQ ID NO: 37;
And, the following three light chain variable region CDRs:
   LCDR1 having an amino acid sequence as shown in SEQ ID NO: 44;
   LCDR2 having an amino acid sequence as shown in SEQ ID NO: 47; and
   LCDR3 having an amino acid sequence as shown in SEQ ID NO: 48.

In another preferred embodiment, the LRRC15 antigen binding domain comprises a heavy chain variable region having at least 80% sequence identity to the amino acid sequence shown in SEQ ID NO: 1, 2, 3, or 4, and a light chain variable region having at least 80% sequence identity to the amino acid sequence shown in SEQ ID NO: 5, 6, 7, or 8.

In another preferred embodiment, the LRRC15 antigen binding domain comprises a heavy chain variable region as shown in SEQ ID NO: 1, and a light chain variable region as shown in SEQ ID NO: 5.

In another preferred embodiment, the LRRC15 antigen binding domain comprises a heavy chain variable region as shown in SEQ ID NO: 2, and a light chain variable region as shown in SEQ ID NO: 6.

In another preferred embodiment, the LRRC15 antigen binding domain comprises a heavy chain variable region as shown in SEQ ID NO: 3, and a light chain variable region as shown in SEQ ID NO: 7.

In another preferred embodiment, the LRRC15 antigen binding domain comprises a heavy chain variable region as shown in SEQ ID NO: 4, and a light chain variable region as shown in SEQ ID NO: 8.

In another preferred embodiment, the LRRC15 antigen binding domain comprises a heavy chain variable region as shown in SEQ ID NO: 53, and a light chain variable region as shown in SEQ ID NO: 54.

In another preferred embodiment, the LRRC15 antigen binding domain is selected from the group consisting of: scFv, Fab, and a combination thereof.

In another preferred embodiment, the LRRC15 antigen binding domain is scFv.

In another preferred embodiment, the LRRC15 antigen binding domain is Fab, which comprises a heavy chain variable region as shown in SEQ ID NO: 1, 2, 3, 4 or 53, and a light chain variable region as shown in SEQ ID NO: 5, 6, 7, 8 or 54; and

A heavy chain constant region CH1 as shown in SEQ ID NO: 26 or 28, and a light chain constant region CL as shown in SEQ ID NO: 30 or 31.

In another preferred embodiment, the second targeting domain is selected from the group consisting of: CD3 antigen binding domain, CD28 antigen binding domain, CD40 antigen binding domain, CD137 antigen binding domain, CD40L sequence, and CD137L sequence.

In another preferred embodiment, the third targeting domain is selected from the group consisting of: CD3 antigen binding domain, CD28 antigen binding domain, CD40 antigen binding domain, CD137 antigen binding domain, CD40L sequence, and CD137L sequence.

In another preferred embodiment, the multi-specific T cell engager further comprises an Fc fragment.

In another preferred embodiment, the Fc fragment is derived from IgG1 or IgG4.

In another preferred embodiment, the Fc fragment is an Fc fragment derived from IgG1, which has an amino acid sequence as shown in SEQ ID NO: 27.

In another preferred embodiment, the Fc fragment derived from IgG1 has mutations selected from the group consisting of:
N297A,
L234F/L235E/P331S,
Y349C/K370E/K409D/K439E,
S354C/D356K/E357K/D399K,
S354C/T366W,
Y349C/T366S/L368A/Y407V,
L234F/L235E/P331S/Y349C/K370E/K409D/K439E,
L234F/L23 5E/P3 31S/S 3 54C/D3 56K/E3 5 7K/D3 99K,
L234F/L235E/P331S/S354C/T366W,
L234F/L235E/P331S/Y349C/T366S/L368A/Y407V,
N297A/Y349C/K370E/K409D/K439E,
N297A/S 3 54C/D3 56K/E3 57K/D3 99K,
N297A/S354C/T366W,
N297A/Y349C/T366S/L368A/Y407V; and/or
S267E, S267E/G236D, S267E/S239D, S267E/L328F.

In another preferred embodiment, the Fc fragment has an amino acid sequence as shown in any one of SEQ ID NOs: 57-60.

In another preferred embodiment, the Fc fragment is an Fc fragment derived from IgG4, which has an amino acid sequence as shown in SEQ ID NO: 29.

In another preferred embodiment, the Fc fragment derived from IgG4 has mutations selected from the group consisting of:
Y349C/K370E/R409D/K439E,
S354C/E356K/E357K/D399K,
S354C/T366W,
Y349C/T366S/L368A/Y407V; and/or
S228P/S267E,
S228P/S267E/G236D,
S228P/S267E/S239D,
S228P/S267E/L328F.

In another preferred embodiment, the multi-specific T cell engager is a bispecific/trispecific T cell engager.

In another preferred embodiment, the multi-specific T cell engager is a bispecific T cell engager.

In another preferred embodiment, the bispecific T cell engager comprises:
A first targeting domain comprising one or more LRRC15 antigen binding domains; and
A second targeting domain, wherein the second targeting domain is a CD3 antigen binding domain.

In another preferred embodiment, the bispecific T cell engager comprises:
A first targeting domain comprising one or more LRRC15 antigen binding domains; and
A second targeting domain, wherein the second targeting domain is a CD40 antigen binding domain.

In another preferred embodiment, the bispecific T cell engager comprises:
A first targeting domain comprising one or more LRRC15 antigen binding domains; and
A second targeting domain, wherein the second targeting domain is a CD137 antigen binding domain.

In another preferred embodiment, the bispecific T cell engager comprises:
A first targeting domain comprising one or more LRRC15 antigen binding domains; and
A second targeting domain, wherein the second targeting domain is one or more CD40L sequences.

In another preferred embodiment, the bispecific T cell engager comprises:
A first targeting domain comprising one or more LRRC15 antigen binding domains; and
A second targeting domain, wherein the second targeting domain is one or more CD137L sequences.

In another preferred embodiment, the multi-specific T cell engager is a trispecific T cell engager.

In another preferred embodiment, the trispecific T cell engager comprises:
A first targeting domain comprising one or more LRRC15 antigen binding domains;
A second targeting domain, wherein the second targeting domain is a CD3 antigen binding domain; and
A third targeting domain, wherein the third targeting domain is a CD28 antigen binding domain.

In another preferred embodiment, the multi-specific T cell engager is a trispecific T cell engager.

In another preferred embodiment, the trispecific T cell engager comprises:
A first targeting domain comprising one or more LRRC15 antigen binding domains;
A second targeting domain, wherein the second targeting domain is a CD3 antigen binding domain; and
A third targeting domain, wherein the third targeting domain is a CD40 antigen binding domain.

In another preferred embodiment, the multi-specific T cell engager is a trispecific T cell engager.

In another preferred embodiment, the trispecific T cell engager comprises:
A first targeting domain comprising one or more LRRC15 antigen binding domains;
A second targeting domain, wherein the second targeting domain is a CD3 antigen binding domain; and
A third targeting domain, wherein the third targeting domain is a CD137 antigen binding domain.

In another preferred embodiment, the multi-specific T cell engager is a trispecific T cell engager.

In another preferred embodiment, the trispecific T cell engager comprises:
A first targeting domain comprising one or more LRRC15 antigen binding domains;
A second targeting domain, wherein the second targeting domain is a CD3 antigen binding domain; and

A third targeting domain, wherein the third targeting domain is a CD40L sequence.

In another preferred embodiment, the trispecific T cell engager comprises:
A first targeting domain comprising one or more LRRC15 antigen binding domains;
A second targeting domain, wherein the second targeting domain is a CD3 antigen binding domain; and
A third targeting domain, wherein the third targeting domain is a CD137L sequence.

In another preferred embodiment, the CD3 antigen binding domain comprises a heavy chain variable region having at least 80% sequence identity to the amino acid sequence shown in SEQ ID NO: 11, 9, 10, or 12, and a light chain variable region having at least 80% sequence identity to the amino acid sequence shown in SEQ ID NO: 13.

In another preferred embodiment, the CD3 antigen binding domain is selected from the group consisting of: scFv, Fab, and a combination thereof.

In another preferred embodiment, the CD28 antigen binding domain comprises a heavy chain variable region having at least 80% sequence identity to the amino acid sequence shown in SEQ ID NO: 14, and a light chain variable region having at least 80% sequence identity to the amino acid sequence shown in SEQ ID NO: 15.

In another preferred embodiment, the CD28 antigen binding domain is selected from the group consisting of: scFv, Fab, and a combination thereof.

In another preferred embodiment, the CD40 antigen binding domain comprises a heavy chain variable region having at least 80% sequence identity to the amino acid sequence shown in SEQ ID NO: 16, and a light chain variable region having at least 80% sequence identity to the amino acid sequence shown in SEQ ID NO: 17.

In another preferred embodiment, the CD40 antigen binding domain comprises a heavy chain variable region with the amino acid sequence shown in SEQ ID NO: 55, and a light chain variable region having at least 80% sequence identity to the amino acid sequence shown in SEQ ID NO: 56.

In another preferred embodiment, the CD40 antigen binding domain is selected from the group consisting of: scFv, Fab, and a combination thereof.

In another preferred embodiment, the CD137 antigen binding domain comprises a heavy chain variable region having at least 80% sequence identity to the amino acid sequence shown in SEQ ID NO: 18 or 20, and a light chain variable region having at least 80% sequence identity to the amino acid sequence shown in SEQ ID NO: 19 or 21.

In another preferred embodiment, the CD137 antigen binding domain is selected from the group consisting of: scFv, Fab, and a combination thereof.

In another preferred embodiment, the CD40L sequence is the RBD receptor binding domain sequence of CD40L, which has the amino acid sequence shown in SEQ ID NO: 23.

In another preferred embodiment, the CD137L sequence is the RBD receptor binding domain sequence of CD137L, which has the amino acid sequence shown in SEQ ID NO: 25.

In another preferred embodiment, the multi-specific T cell engager has a structure as shown in Formula I: 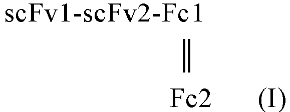
wherein "-" each independently represents a peptide bond or a linker peptide; "||" represents a linking bond between the polypeptide chains.
scFv1 is the first targeting domain, and the scFv1 is an anti-LRRC15 scFv;
scFv2 is the second targeting domain, and the scFv2 is an anti-CD3 scFv;
Fc1 and Fc2 each independently represent an Fc fragment.

In another preferred embodiment, the anti-LRRC15 scFv comprises a heavy chain variable region as shown in SEQ ID NO: 1, 2, 3, 4 or 53, and a light chain variable region as shown in SEQ ID NO: 5, 6, 7, 8 or 54.

In another preferred embodiment, the LRRC15 scFv comprises a heavy chain variable region as shown in SEQ ID NO: 53, and a light chain variable region as shown in SEQ ID NO: 54.

In another preferred embodiment, the anti-CD3 scFv comprises a heavy chain variable region as shown in SEQ ID NO: 11, 9, 10, or 12, and a light chain variable region as shown in SEQ ID NO: 13.

In another preferred embodiment, the anti-CD3 scFv comprises a heavy chain variable region as shown in SEQ ID NO: 11, and a light chain variable region as shown in SEQ ID NO: 13.

In another preferred embodiment, the Fc fragment is derived from IgG1 or IgG4.

In another preferred embodiment, the Fc fragment is derived from IgG1.

In another preferred embodiment, the Fc fragment has an amino acid sequence as shown in SEQ ID NO: 57 or 58.

In another preferred embodiment, the Fc1 has an amino acid sequence as shown in SEQ ID NO: 57, and the Fc2 has an amino acid sequence as shown in SEQ ID NO: 58.

In another preferred embodiment, the amino acid sequence of "scFvl-scFv2-Fcl" is as shown in SEQ ID NO: 64; the amino acid sequence of "Fc2" is as shown in SEQ ID NO: 58.

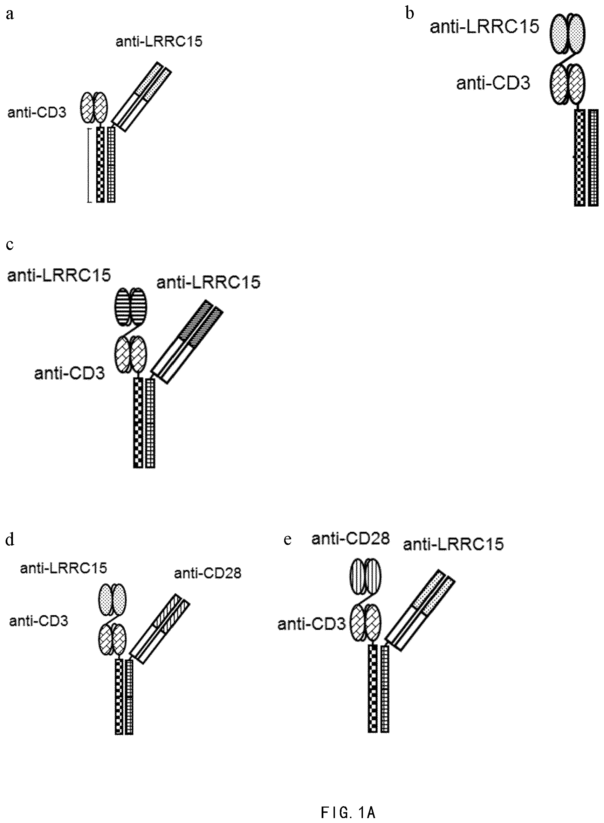

In another preferred embodiment, the multi-specific T cell engager has a structure as shown in Formula II (Figure 1A-c): 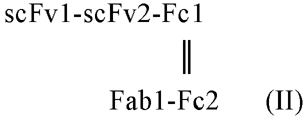
wherein "-" each independently represents a peptide bond or a linker peptide; "||" represents a linking bond between the polypeptide chains.
scFv1 and Fab1 are the first targeting domains, the scFv1 is an anti-LRRC15 scFv, and the Fab1 is an anti-LRRC15 Fab;
scFv2 is the second targeting domain, and the scFv2 is an anti-CD3 scFv;
Fc1 and Fc2 each independently represent an Fc fragment.

In another preferred embodiment, the anti-LRRC15 scFv comprises a heavy chain variable region as shown in SEQ ID NO: 1, 2, 3, 4 or 53, and a light chain variable region as shown in SEQ ID NO: 5, 6, 7, 8 or 54.

In another preferred embodiment, the anti-LRRC15 Fab comprises a heavy chain variable region as shown in SEQ ID NO: 1, 2, 3, 4 or 53, and a light chain variable region as shown in SEQ ID NO: 5, 6, 7, 8 or 54.

In another preferred embodiment, the LRRC15 scFv comprises a heavy chain variable region as shown in SEQ ID NO: 53, and a light chain variable region as shown in SEQ ID NO: 54.

In another preferred embodiment, the anti-LRRC15 Fab comprises a heavy chain variable region as shown in SEQ ID NO: 2, and a light chain variable region as shown in SEQ ID NO: 6.

In another preferred embodiment, the anti-CD3 scFv comprises a heavy chain variable region as shown in SEQ ID NO: 11, 9, 10, or 12, and a light chain variable region as shown in SEQ ID NO: 13.

In another preferred embodiment, the anti-CD3 scFv comprises a heavy chain variable region as shown in SEQ ID NO: 11, and a light chain variable region as shown in SEQ ID NO: 13.

In another preferred embodiment, the Fc fragment is derived from IgG1 or IgG4.

In another preferred embodiment, the Fc fragment is derived from IgG1.

In another preferred embodiment, the Fc fragment has an amino acid sequence as shown in SEQ ID NO: 57 or 58.

In another preferred embodiment, the Fc1 has an amino acid sequence as shown in SEQ ID NO: 57, and the Fc2 has an amino acid sequence as shown in SEQ ID NO: 58.

In another preferred embodiment, the amino acid sequence of "scFvl-scFv2-Fcl" is as shown in SEQ ID NO: 64.

In another preferred embodiment, in the "Fab1-Fc2", the amino acid sequence of "HC1(heavy chain)-Fc2" is as shown in SEQ ID NO: 81, and the amino acid sequence of "LC1(light chain)" is as shown in SEQ ID NO: 84.

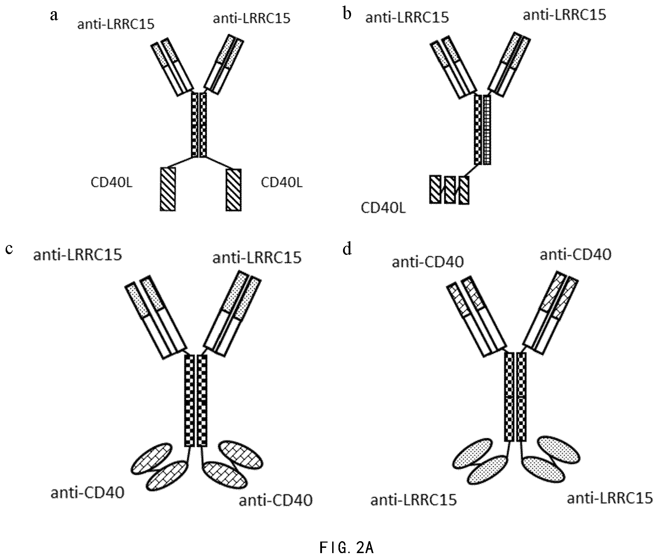

In another preferred embodiment, the multi-specific T cell engager has a structure as shown in Formula III (Figure 2A-c): 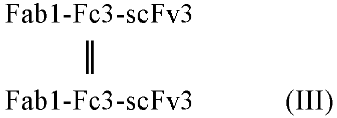
wherein "-" each independently represents a peptide bond or a linker peptide; "||" represents a linking bond between the polypeptide chains.
Fab1 is the first targeting domain, and the Fab1 is an anti-LRRC15 Fab;
scFv3 is the second targeting domain, and the scFv3 is an anti-CD40 scFv;
Fc3 is an Fc fragment.

In another preferred embodiment, the " || " is a disulfide bond.

In another preferred embodiment, the anti-LRRC15 Fab comprises a heavy chain variable region as shown in SEQ ID NO: 1, 2, 3, 4 or 53, and a light chain variable region as shown in SEQ ID NO: 5, 6, 7, 8 or 54.

In another preferred embodiment, the LRRC15 Fab comprises a heavy chain variable region as shown in SEQ ID NO: 1, and a light chain variable region as shown in SEQ ID NO: 5.

In another preferred embodiment, the anti-CD40 scFv comprises a heavy chain variable region as shown in SEQ ID NO: 16, and a light chain variable region as shown in SEQ ID NO: 17.

In another preferred embodiment, the Fc fragment is derived from IgG1 or IgG4.

In another preferred embodiment, the Fc fragment is derived from IgG1.

In another preferred embodiment, the Fc fragment has an amino acid sequence as shown in SEQ ID NO: 59 or 60.

In another preferred embodiment, in the "Fab1-Fc3-scFv3", the amino acid sequence of "HC1(heavy chain)-Fc3-scFv3" is as shown in SEQ ID NO: 70, and the amino acid sequence of "LC1(light chain)" is as shown in SEQ ID NO: 83.

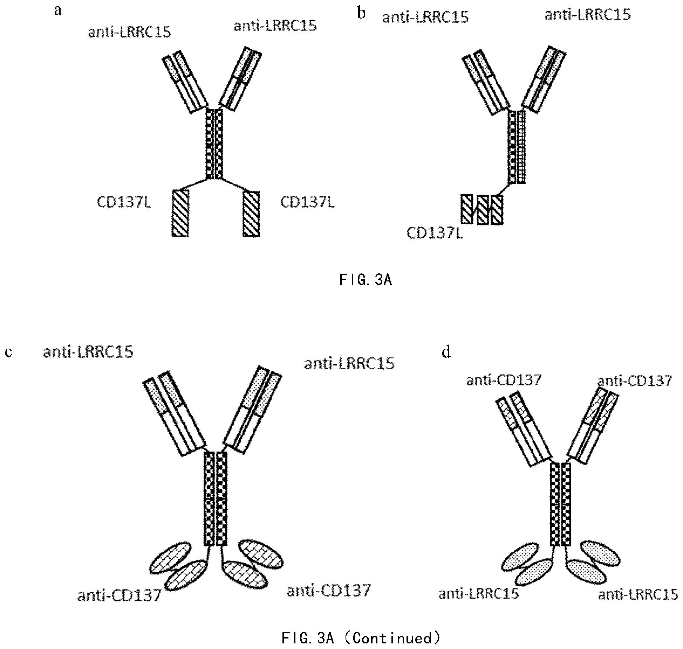

In another preferred embodiment, the multi-specific T cell engager has a structure as shown in Formula IV (Figure 3A-c): 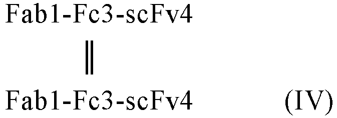
wherein "-" each independently represents a peptide bond or a linker peptide; "||" represents a linking bond between the polypeptide chains.
Fab1 is the first targeting domain, and the Fab1 is an anti-LRRC15 Fab;
scFv4 is the second targeting domain, and the scFv4 is an anti-CD137 scFv;
Fc3 is an Fc fragment.

In another preferred embodiment, the " ||" is a disulfide bond.

In another preferred embodiment, the anti-LRRC15 Fab comprises a heavy chain variable region as shown in SEQ ID NO: 1, 2, 3, 4 or 53, and a light chain variable region as shown in SEQ ID NO: 5, 6, 7, 8 or 54.

In another preferred embodiment, the LRRC15 Fab comprises a heavy chain variable region as shown in SEQ ID NO: 1, and a light chain variable region as shown in SEQ ID NO: 5.

In another preferred embodiment, the anti-CD137 scFv comprises a heavy chain variable region as shown in SEQ ID NO: 18 or 20, and a light chain variable region as shown in SEQ ID NO: 19 or 21.

In another preferred embodiment, the anti-CD137 scFv comprises a heavy chain variable region as shown in SEQ ID NO: 20, and a light chain variable region as shown in SEQ ID NO: 21.

In another preferred embodiment, the Fc fragment is derived from IgG1 or IgG4.

In another preferred embodiment, the Fc fragment is derived from IgG1.

In another preferred embodiment, the Fc fragment has an amino acid sequence as shown in SEQ ID NO: 59 or 60.

In another preferred embodiment, in the "Fab1-Fc3-scFv4", the amino acid sequence of "HC1(heavy chain)-Fc3-scFv4" is as shown in SEQ ID NO: 77, and the amino acid sequence of "LC1(light chain)" is as shown in SEQ ID NO: 83.

In the second aspect of the present invention, it provides an antibody or antigen-binding fragment thereof against LRRC15, wherein the antibody comprises the following three heavy chain variable region CDRs:
HCDR1 having an amino acid sequence as shown in SEQ ID NO: 32, 35, 38, or 41;
HCDR2 having an amino acid sequence as shown in SEQ ID NO: 33, 36, 39, or 42; and
HCDR3 having an amino acid sequence as shown in SEQ ID NO: 34, 37, 40, or 43;
and, the following three light chain variable region CDRs:
   LCDR1 having an amino acid sequence as shown in SEQ ID NO: 44 or 49;
   LCDR2 having an amino acid sequence as shown in SEQ ID NO: 45, 47, or 50; and
   LCDR3 having an amino acid sequence as shown in SEQ ID NO: 46, 48, 51, or 52.

In another preferred embodiment, the antibody or antigen-binding fragment thereof against LRRC15 comprises three heavy chain variable region CDRs (HCDRs) and three light chain variable region CDRs (LCDRs) selected from the group consisting of:

| Antibody Name | SEQ ID NO of HCDR1 | SEQ ID NO of HCDR2 | SEQ ID NO of HCDR3 | SEQ ID NO of LCDR1 | SEQ ID NO of LCDR2 | SEQ ID NO of LCDR3 |
|---|---|---|---|---|---|---|
| L15-06/ L15-06C | 32 | 33 | 34 | 44 | 45 | 46 |
| L15-08 | 35 | 36 | 37 | 44 | 47 | 48 |
| L15-03 | 38 | 39 | 40 | 49 | 50 | 51 |
| L15-07 | 41 | 42 | 43 | 49 | 50 | 52 |

In another preferred embodiment, the antibody or antigen-binding fragment thereof against LRRC15 comprises a heavy chain variable region having at least 80% sequence identity to the amino acid sequence as shown in SEQ ID NO: 1, 2, 3, or 4, and a light chain variable region having at least 80% sequence identity to the amino acid sequence as shown in SEQ ID NO: 5, 6, 7, or 8.

In another preferred embodiment, the antibody or antigen-binding fragment thereof against LRRC 15 comprises a heavy chain variable region as shown in SEQ ID NO: 1, and a light chain variable region as shown in SEQ ID NO: 5.

In another preferred embodiment, the antibody or antigen-binding fragment thereof against LRRC 15 comprises a heavy chain variable region as shown in SEQ ID NO: 2, and a light chain variable region as shown in SEQ ID NO: 6.

In another preferred embodiment, the antibody or antigen-binding fragment thereof against LRRC 15 comprises a heavy chain variable region as shown in SEQ ID NO: 3, and a light chain variable region as shown in SEQ ID NO: 7.

In another preferred embodiment, the antibody or antigen-binding fragment thereof against LRRC 15 comprises a heavy chain variable region as shown in SEQ ID NO: 4, and a light chain variable region as shown in SEQ ID NO: 8.

In another preferred embodiment, the antibody or antigen-binding fragment thereof against LRRC15 comprises a heavy chain variable region as shown in SEQ ID NO: 53, and a light chain variable region as shown in SEQ ID NO: 54.

In another preferred embodiment, the antibody or antigen-binding fragment thereof is a human, mouse, humanized or chimeric antibody.

In another preferred embodiment, the antibody or antigen-binding fragment thereof is a human antibody.

In the third aspect of the present invention, it provides a polynucleotide encoding the multi-specific T cell engager according to the first aspect of the present invention or the antibody according to the second aspect of the present invention.

In the fourth aspect of the present invention, it provides an expression vector comprising the polynucleotide according to the third aspect of the present invention.

In another preferred embodiment, the expression vector includes a prokaryotic expression vector and a eukaryotic expression vector.

In the fifth aspect of the present invention, it provides a host cell comprising the expression vector according to the fourth aspect of the present invention, or the polynucleotide according to the third aspect of the present invention integrated into the genome thereof.

In another preferred embodiment, the host cell includes a prokaryotic cell or a eukaryotic cell.

In another preferred embodiment, the host cell is selected from the group consisting of: *Escherichia coli,* yeast cells, HEK 293T cells, CHO cells.

In the sixth aspect of the present invention, it provides a use of the multi-specific T cell engager according to the first aspect of the present invention for preparing a medicament for treating cancer/tumor.

In another preferred embodiment, the cancer/tumor is a cancer/tumor with high LRRC15 expression.

In another preferred embodiment, the cancer/tumor includes solid tumors and hematological tumors.

In another preferred embodiment, the cancer/tumor is a solid tumor.

In another preferred embodiment, the cancer/tumor is selected from the group consisting of: breast cancer, head and neck tumors, lung cancer, pancreatic cancer, ovarian cancer, melanoma, malignant glioma, sarcoma, bone cancer, and a combination thereof.

In the seventh aspect of the present invention, it provides an immunoconjugate comprising:
(i) the multi-specific T cell engager according to the first aspect of the present invention, or the antibody or antigen-binding fragment thereof according to the second aspect of the present invention; and
(ii) a coupling moiety selected from the group consisting of: a detectable label, a drug, a toxin, a cytokine, a radionuclide, and an enzyme.

In another preferred embodiment, the conjugate is selected from: fluorescent or luminescent labels, radioactive labels, MRI (magnetic resonance imaging) or CT (computed tomography) contrast agents, or enzymes capable of generating detectable products, radionuclides, biological toxins, cytokines (such as IL-2, etc.), antibodies, antibody Fc fragments, antibody scFv fragments, gold nanoparticles/nanorods, virus particles, liposomes, magnetic nanoparticles, prodrug activating enzymes (e.g., DT-diaphorase (DTD) or biphenyl hydrolase-like protein (BPHL)), chemotherapeutic agents (e.g., cisplatin) or any form of nanoparticles, etc.

In the eighth aspect of the present invention, it provides a pharmaceutical composition comprising: (a) the multi-specific T cell engager according to the first aspect of the present invention, or the antibody or antigen-binding fragment thereof according to the second aspect of the present invention; and (b) a pharmaceutically acceptable carrier.

In another preferred embodiment, the pharmaceutical composition is an injection.

In the ninth aspect of the present invention, it provides a method for treating cancer/tumor, which comprises a step of administering the multi-specific T cell engager according to the first aspect of the present invention to a subject in need thereof.

In another preferred embodiment, the subject in need thereof is a human or a non-human mammal.

In another preferred embodiment, the cancer/tumor is a cancer/tumor with high LRRC15 expression.

In another preferred embodiment, the cancer/tumor includes solid tumors and hematologicaltumors.

In another preferred embodiment, the cancer/tumor is a solid tumor.

In another preferred embodiment, the cancer/tumor is selected from the group consisting of: breast cancer, head and neck tumors, lung cancer, pancreatic cancer, ovarian cancer, melanoma, malignant glioma, sarcoma, bone cancer, and a combination thereof.

It should be understood that within the scope of the present invention, the above technical features of the present invention and the technical features specifically described below (such as embodiments) can be combined with each other to form new or preferred technical solutions. Due to space limitations, they are not listed here one by one.

### DESCRIPTION OF DRWAINGS

Figure 1A shows three structural forms of bispecific antibodies composed of LRRC15 antigen binding domain and CD3 antigen binding domain (a-c); and two structural forms of trispecific antibodies composed of LRRC15 antigen binding domain, CD3 antigen binding domain, and CD28 antigen binding domain (d-e);
   Among them, the antibody structure a is composed of anti-CD3 scFv and anti-LRRC15 Fab (CH1-VH + CK-VL), wherein IgG1 Fc forms a heterodimer through charge pairs or knob and hole mutations; the antibody structure b is formed by anti-LRRC15 scFv and anti-CD3 scFv (scFv-scFv-Fc×Fc); wherein IgG1 Fc forms a heterodimer through charge pairs or knob and hole mutations; the molecular structure c is formed by anti-LRRC15 scFv, anti-CD3 scFv and anti-LRRC15 Fab (scFv-scFv-Fc×Fab-Fc), wherein IgG1 Fc forms a heterodimer through charge pairs or knob and hole mutations; the molecular structure d is formed by anti-LRRC15 scFv, anti-CD3 scFv and anti-CD28 Fab (scFv-scFv-Fc×Fab-Fc), wherein IgG1 Fc forms a heterodimer through charge pairs or knob and hole mutations; the molecular structure e is formed by anti-CD28 scFv, anti-CD3 scFv and anti-LRRC15 Fab (scFv-scFv-Fc×Fab-Fc), wherein IgG1 Fc forms a heterodimer through charge pairs or knob and hole mutations.
Figure 1B shows an exemplary bispecific antibody of structure b in Figure 1A, named 153-L06C.
Figure 1C shows an exemplary bispecific antibody of structure c in Figure 1A, named 315-L06C08.
Figure 1D shows an exemplary bispecific antibody of structure a in Figure 1A, named 315-L06.
Figure 1E shows an exemplary trispecific antibody of structure e in Figure 1A, named 28315-L06.
Figure 2A shows four structural forms of bispecific antibodies composed of LRRC15 antigen binding domain and CD40 antigen binding domain;
   Among them, the molecular structure a is formed by anti-LRRC15 antibody and CD40L (IgG-RBD×IgG-RBD); the molecular structure b is formed by anti-LRRC15 antibody and CD40L (IgG-trimeric RBD×Fab-Fc); the molecular structure c is formed by anti-LRRC15 antibody and anti-CD40 scFv (IgG-scFv×IgG-scFv); the molecular structure d is formed by anti-CD40 antibody and anti-LRRC15 scFv (IgG-scFv×IgG-scFv).

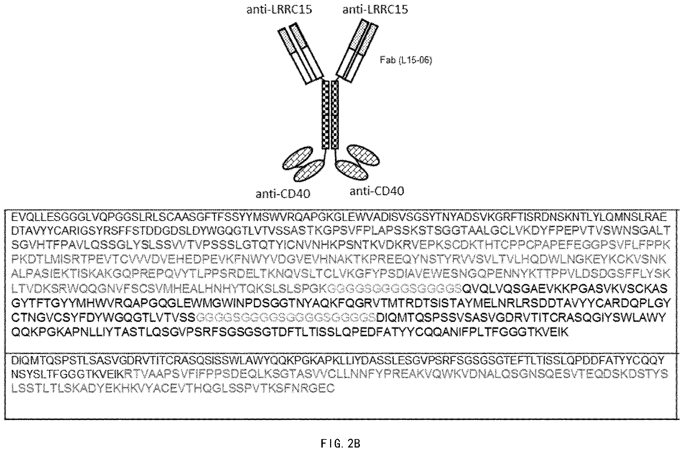

Figure 2B shows an exemplary bispecific antibody of structure c in Figure 2A, named 1540-L06.

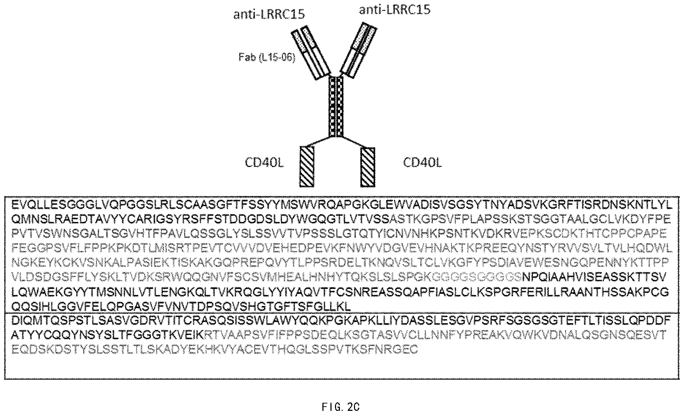

Figure 2C shows an exemplary bispecific antibody of structure a in Figure 2A, named 1540L-L06.

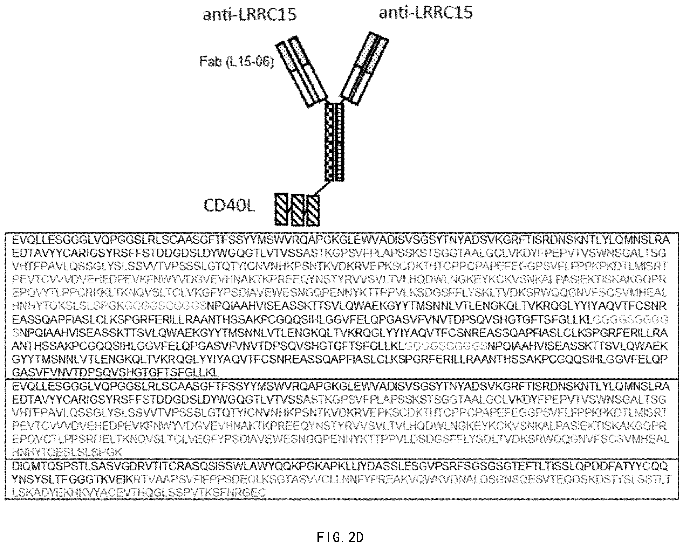

Figure 2D shows an exemplary bispecific antibody of structure b in Figure 2A, named 1540TL-L06.

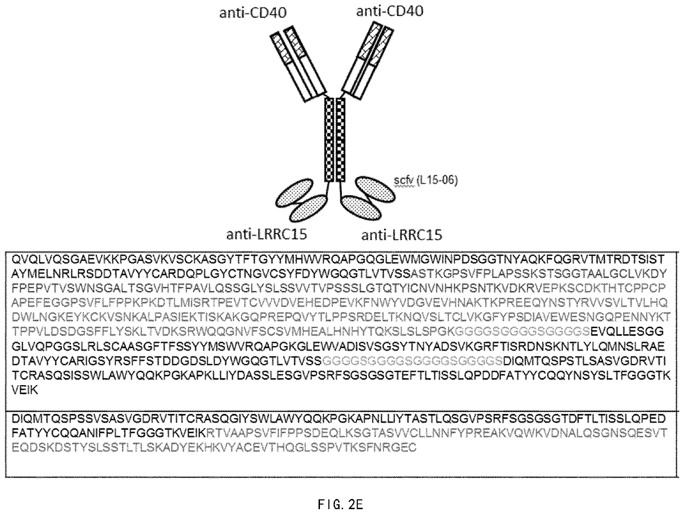

Figure 2E shows an exemplary bispecific antibody of structure d in Figure 2A, named 4015-L06.
Figure 3A shows four structural forms of bispecific antibodies composed of LRRC15 antigen binding domain and CD137 antigen binding domain;
   Among them, the molecular structure a is formed by anti-LRRC15 antibody and CD137L (IgG-RBD×IgG-RBD); the molecular structure b is formed by anti-LRRC15 antibody and CD137L (IgG-trimeric RBD×Fab-Fc); the molecular structure c is formed by anti-LRRC15 antibody and anti-CD137 scFv (IgG-scFv×IgG-scFv); the molecular structure d is formed by anti-CD137 antibody and anti-LRRC15 scFv (IgG-scFv×IgG-scFv).

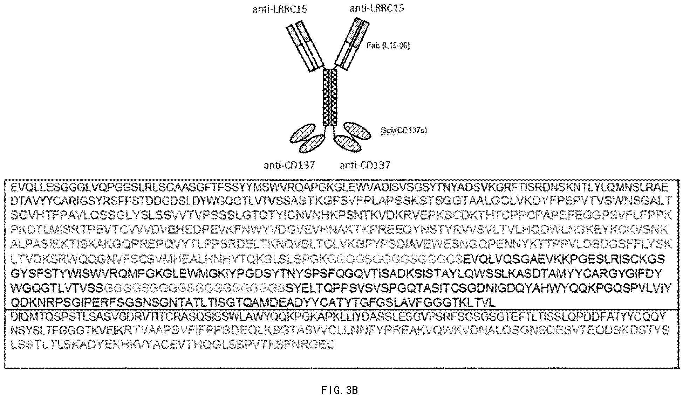

Figure 3B shows an exemplary bispecific antibody of structure c in Figure 3A, named 15BB-L06.

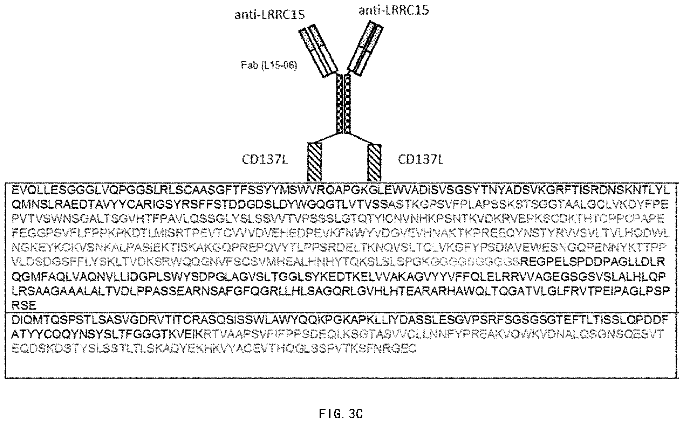

Figure 3C shows an exemplary bispecific antibody of structure a in Figure 3A, named 15BBL-L06.

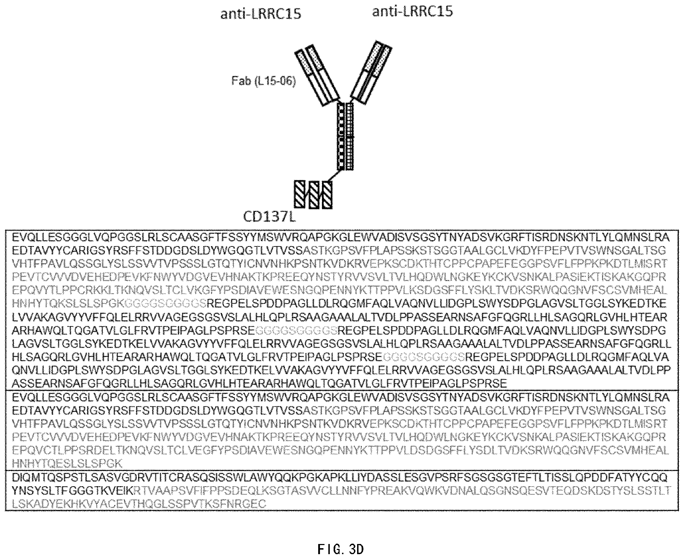

Figure 3D shows an exemplary bispecific antibody of structure b in Figure 3A, named 15BBTL-L06.

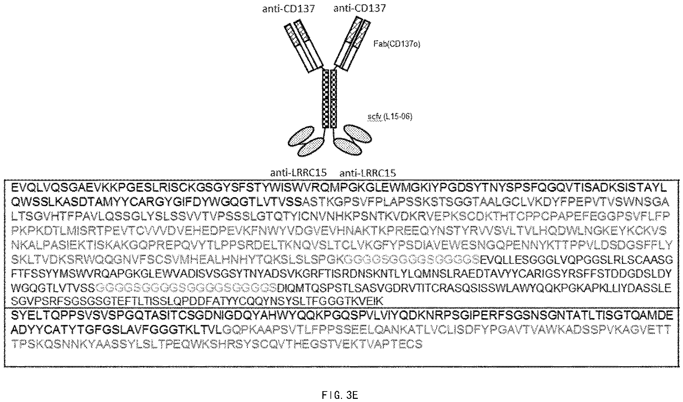

Figure 3E shows an exemplary bispecific antibody of structure d in Figure 3A, named BB15-L06.

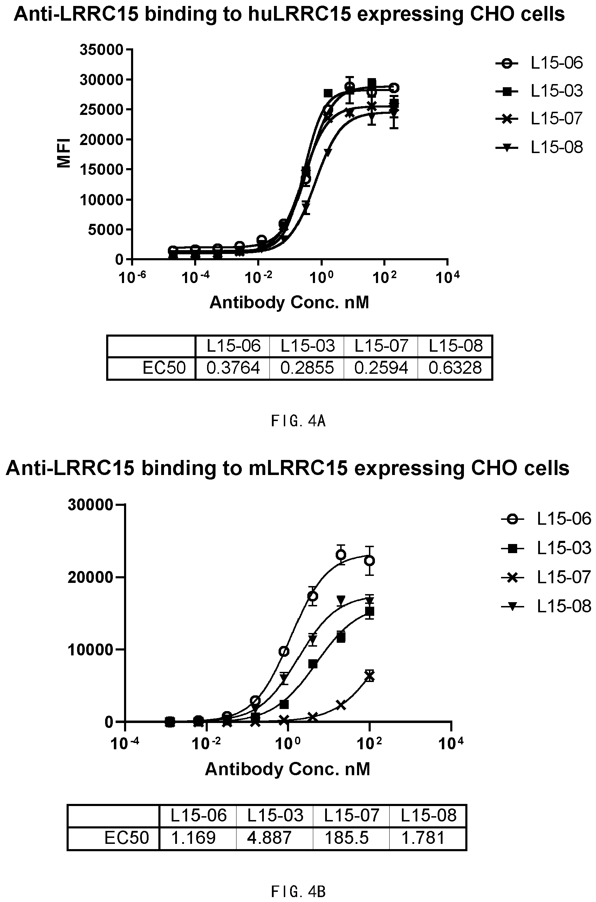

Figure 4A shows FACS detection of the binding of anti-LRRC15 antibody to a cell line expressing human LRRC15.
Figure 4B shows FACS detection of the binding of anti-LRRC15 antibody to a cell line expressing mouse LRRC15.

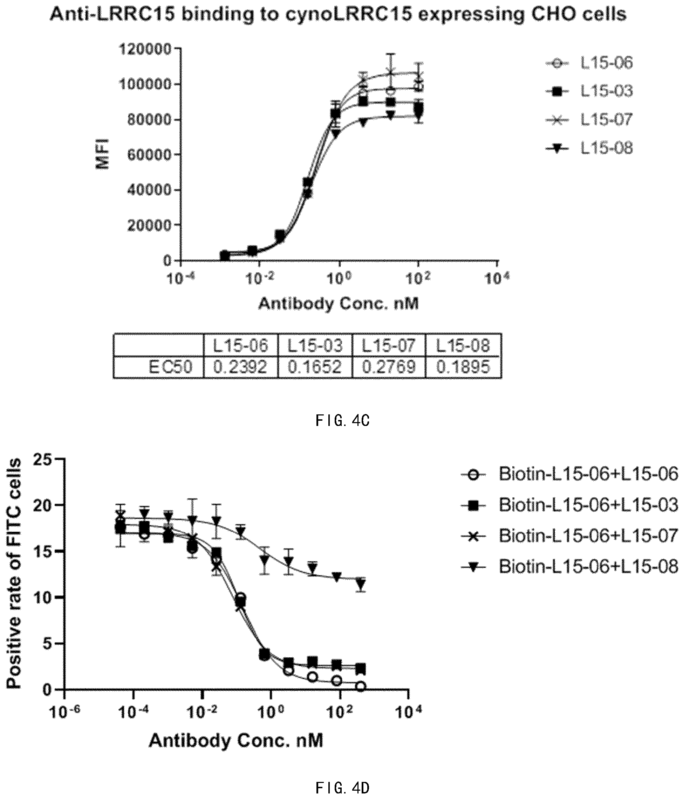

Figure 4C shows FACS detection of the binding of anti-LRRC15 antibody to a cell line expressing cynomolgus LRRC15.
Figure 4D shows FACS detection of the competitive binding of LRRC15 antibodies to a cell line expressing human LRRC15.

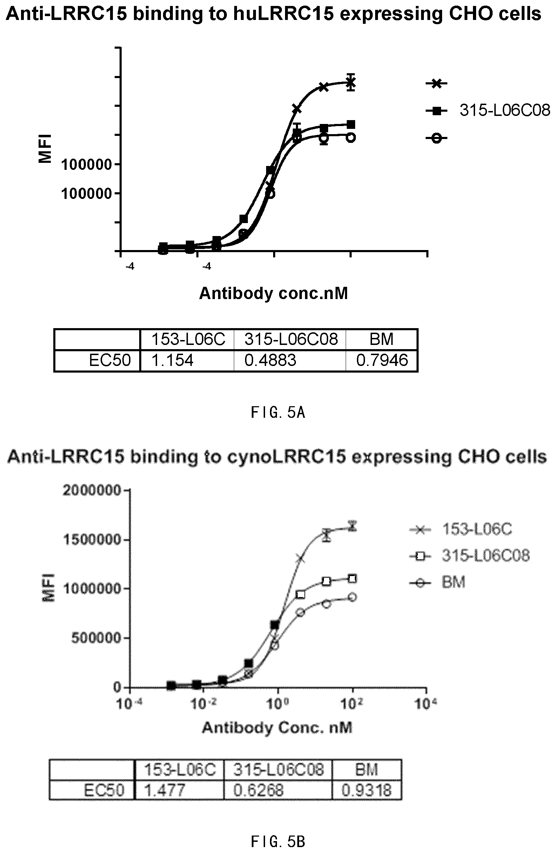

Figure 5A shows FACS detection of the binding of bispecific antibodies targeting LRRC15 and CD3 (153-L06C and 315-L06C08) to a cell line expressing human LRRC15.
Figure 5B shows FACS detection of the binding of bispecific antibodies targeting LRRC15 and CD3 (153-L06C and 315-L06C08) to a cell line expressing cynomolgus LRRC15.

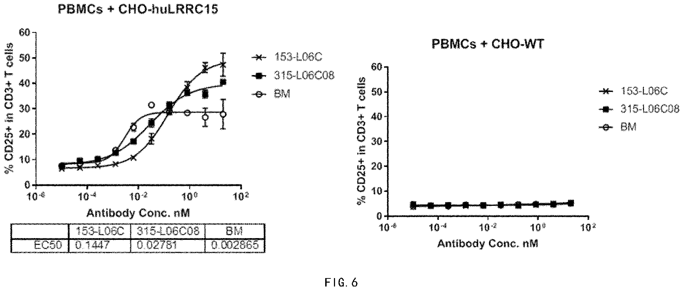

Figure 6 shows the curve of the variation of the proportion of T cells expressing CD25 after stimulation of PBMC with bispecific antibodies (153-L06C; 315-L06C08) according to different antibody concentrations.

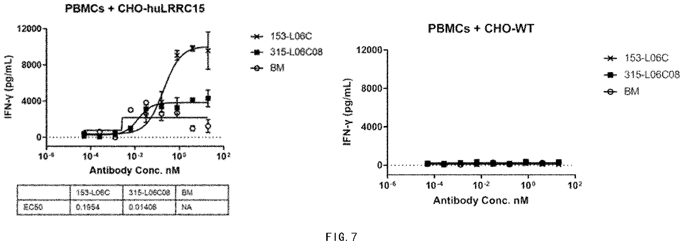

Figure 7 shows the curve of the variation of the IFN-y released by PBMC after stimulation of PBMC with bispecific antibodies (153-L06C; 315-L06C08) according to different antibody concentrations.

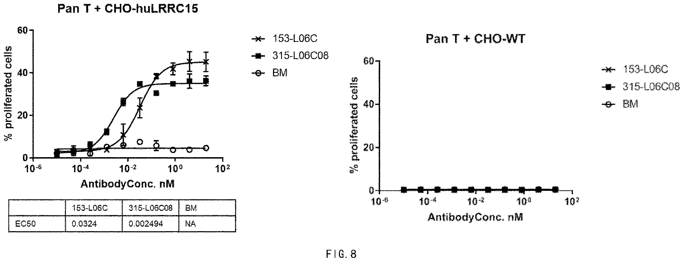

Figure 8 shows the curve of the variation of the proportion of proliferating T cells after stimulation of T cells with bispecific antibodies (153-L06C; 315-L06C08) according to different antibody concentrations.

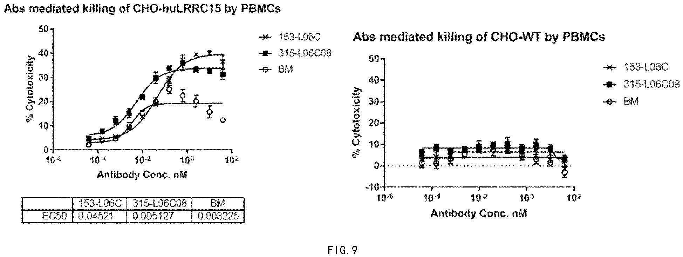

Figure 9 shows the curve of the variation of the killing ratio of target cells after stimulation of PBMC with bispecific antibodies (153-L06C; 315-L06C08) according to different antibody concentrations.

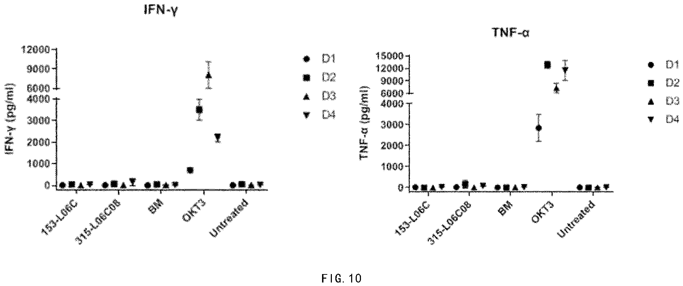

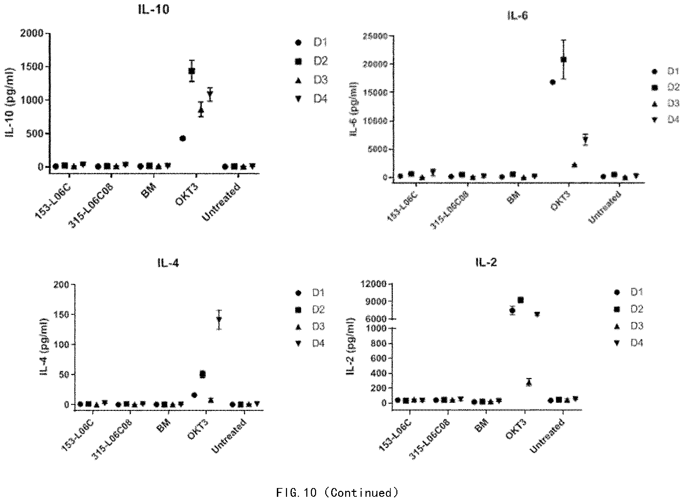

Figure 10 shows the results of the stimulation of PBMC with bispecific antibodies (153-L06C; 315-L06C08) to release cytokines.

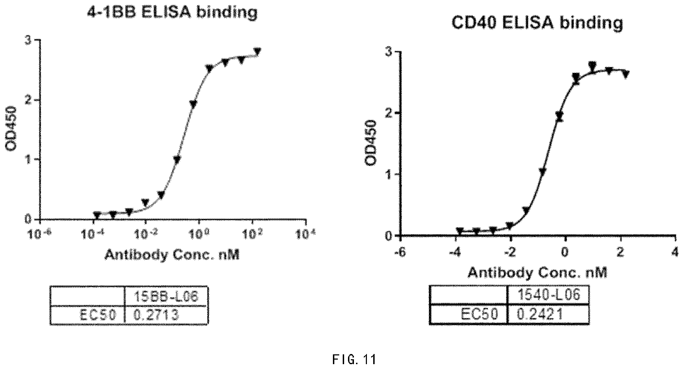

Figure 11 shows the binding curve of the bispecific antibody targeting LRRC15 and CD137 (15BB-L06) to 4-1BB protein, and the binding curve of the bispecific antibody targeting LRRC15 and CD40 (1540-L06) to CD40 protein detected by ELISA.

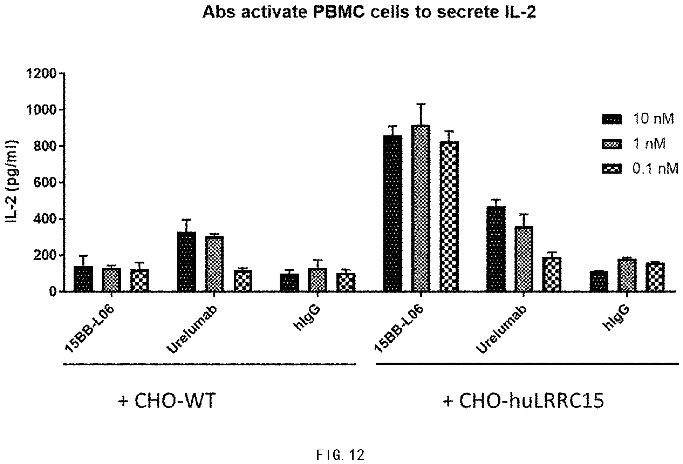

Figure 12 shows the results of the stimulation of PBMC with bispecific antibody (15BB-L06) to release IL-2.

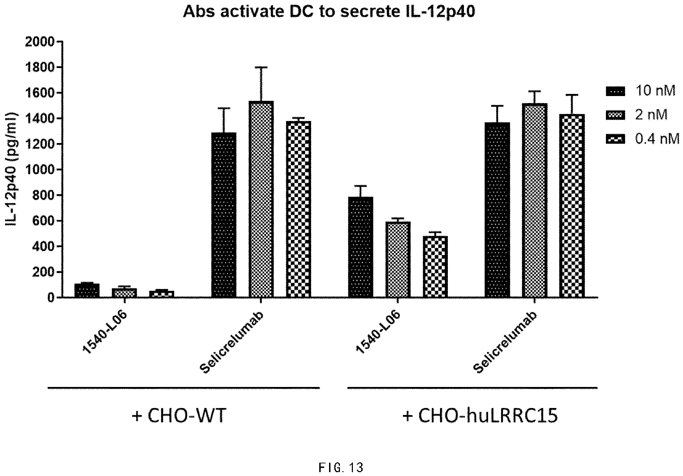

Figure 13 shows the results of the stimulation of DC with bispecific antibody (1540-L06) to release IL-12p40.

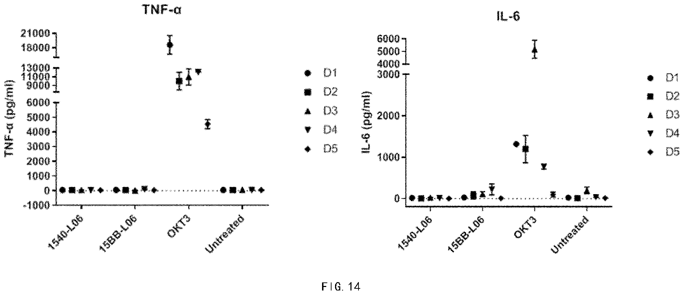

Figure 14 shows the results of the stimulation of PBMC with bispecific antibodies (15BB-L06; 1540-L06) to release cytokines (TNF-α and IL-6).

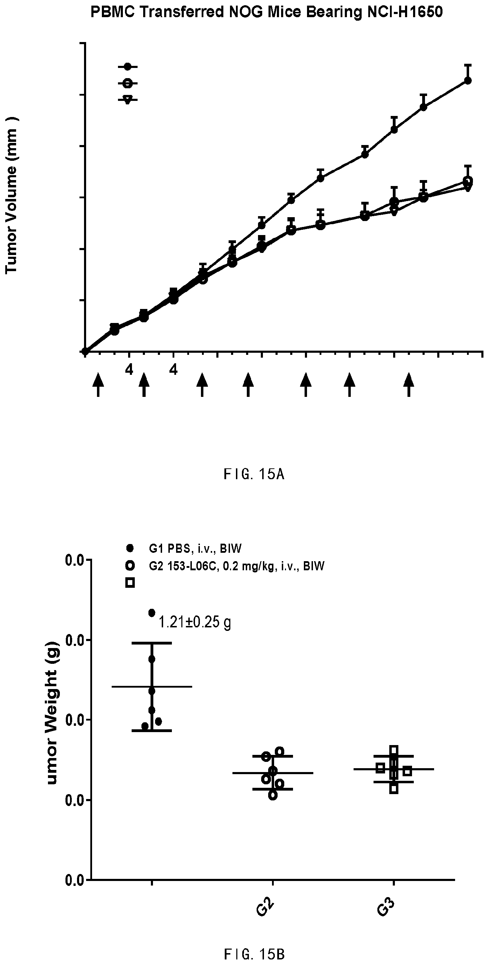

Figure 15A shows the changes in tumor volume in NCI-H1650 PBMC mice during administration.
Figure 15B shows the comparison of tumor weights between groups after administration in NCI-H1650 PBMC mice.

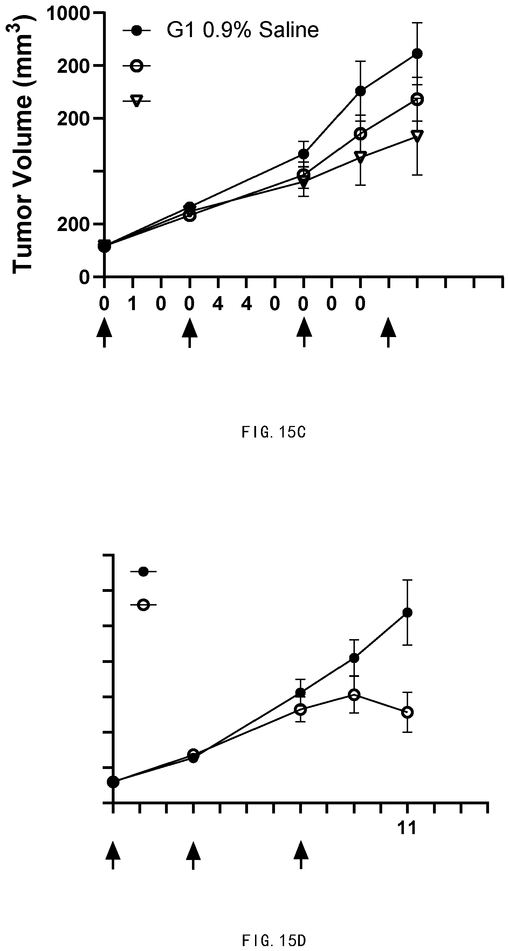

Figure 15C shows the antitumor effect of 153-L06C or 15BB-L06 in MC38 model mice.
Figure 15D shows the antitumor effect of 1540-L06 in MC38 model mice.

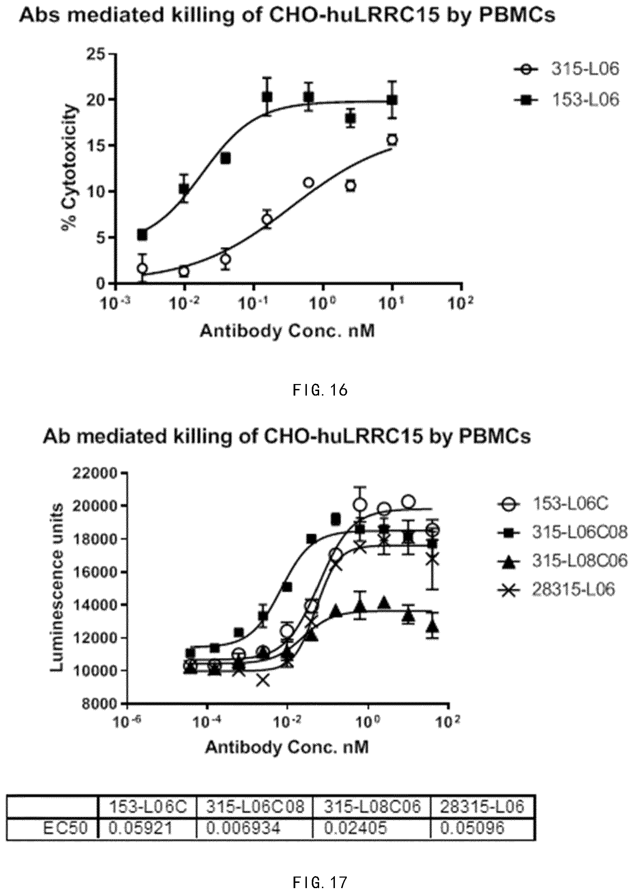

Figure 16 shows a comparison of the killing effects of 153-L06 and 315-L06.
Figure 17 shows a comparison of the killing effects of 153-L06C, 315-L06C08, 315-L08C06 and 28315-L06.

### DETAILED DESCRIPTION

After extensive and in-depth research, the inventors of the present invention unexpectedly developed for the first time a class of multi-specific T cell engagers comprising LRRC15 antigen binding domains. The mult-ispecific T cell engager comprises a first targeting domain that targets the leucine-rich repeat-containing protein 15 (LRRC15) molecules highly expressed on the surface of tumor cells, wherein the first targeting domain is an LRRC15 antibody or antigen-binding fragment thereof, and further comprises a second targeting domain and/or a third targeting domain that binds to the CD molecule on the surface of T cells. The multi-specific T cell engager of the present invention can simultaneously bind to tumor cells and T cells, and can be used as an effective therapeutic agent for tumor treatment, especially for tumors with high LRRC15 expression.

On this basis, the present invention is completed.

As used herein, the term "leucine-rich repeat-containing protein 15" or "LRRC15" refers to the protein encoded by the LRRC15 gene in humans. The leucine-rich repeat (LRR) domain is evolutionarily conserved in proteins associated with innate immunity. LRRC15 is expressed on stromal fibroblasts in many solid tumors (e.g., breast, head and neck, lung, pancreas) and directly on a subset of cancer cells of mesenchymal origin (e.g., sarcoma, melanoma, glioma), while most normal tissues show low or no expression.

As used herein, the terms "CD", "cluster of differentiation", or "cluster of differentiation molecules" refer to cell surface markers that can be used to identify and characterize leukocytes, such as CD3, CD28, and CD40. CD3 is the signaling component of the T cell receptor (TCR) complex. Since T cell activation requires CD3, drugs (usually monoclonal antibodies) that target it are being studied as immunostimulants for the treatment of cancer.

CD28 is the major costimulatory molecule required for the generation of a T cell-mediated immune response. Upon interaction with its ligands CD80 and CD86, CD28 transduces an activating signal, leading to the expression of anti-apoptotic proteins and enhanced synthesis of multiple cytokines, including IL-2. The CD28 costimulatory receptor is present on all T cells. Agonist antibodies targeting CD28 have led to severe adverse events clinically, compared to antibodies targeting other CD28 family members including CTLA-4, PD-1 or their ligands, the latter as checkpoint inhibitors, overcome tumor immune tolerance and can be used for cancer immunotherapy.

CD40 is a cell surface receptor that belongs to the tumor necrosis factor-R (TNF-R) family and is constitutively expressed on APCs such as B cells and dendritic cells. CD40L (CD154) is a type II transmembrane glycoprotein that can be induced and is restricted to cells of the hematopoietic system, such as platelets, granulocytes, and activated T/B/NK cells. CD40L binding to CD40 activates APCs, leading to upregulation of CD80, CD86 and other costimulatory molecules to stimulate CD8+ antigen-specific T cell responses.

The term "Fc fragment" or "Fc" refers to the portion of an antibody that does not have antigen binding activity but was originally observed to be easily crystallized, and hence was named Fc fragment (for fragment crystallizable). This fragment corresponds to the paired CH2 and CH3 domains, and is the portion of the antibody molecule that interacts with effector molecules and cells. The Fc fragments described herein can be derived from IgG1, IgG2, and IgG4 antibodies. For specific purposes, a particular IgG subclass may be preferred. For example, IgG1 is more effective than IgG2 and IgG4 in mediating ADCC and CDC. Therefore, IgG2 Fc may be preferred when effector function is not desired. However, molecules containing IgG2 Fc are generally more difficult to prepare and may be less stable than molecules containing IgG1 Fc. On the other hand, cross-binding to FcyRIIB is critical for enhancing costimulatory signals from CD40 or CD137, and IgG4 has an affinity about 2-fold higher than IgG1 and 10-fold higher than IgG2. Mutations such as S267E and L328F can further enhance the cross-binding of IgG Fc to FcyRIIB. Additionally, the effector function of an antibody can be increased or decreased by introducing one or more mutations into the Fc (see, e.g., Strohl, Curr. Opin. Biotech., 20:685-691, 2009).

As used herein, the term "antibody" or "immunoglobulin" refers to an approximately 150,000 dalton heterotetrameric protein with the same structural features, which is composed of two identical light chains (L) and two identical heavy chains (H). Each light chain is covalently linked to a heavy chain by a disulfide bond, and the number of disulfide bonds between the heavy chains of different immunoglobulin isotypes varies. Each heavy chain and light chain also has intra-chain disulfide bonds at regular intervals. One end of each heavy chain has a variable region (VH), followed by multiple constant regions. Each light chain has a variable region (VL) at one end and a constant region at the other end; the constant region of the light chain is opposite the first constant region of the heavy chain, and the variable region of the light chain is opposite the variable region of the heavy chain. Specific amino acid residues form an interface between the variable regions of the light and heavy chains.

As used herein, the term "variable" means that certain portions of the variable region of an antibody differ in sequence, and it gives rise to the binding and specificity of various specific antibodies for their specific antigens. However, variability is not evenly distributed throughout the antibody variable region. It is concentrated in three segments within the variable regions of the light and heavy chains called complementarity determining regions (CDRs) or hypervariable regions. The more conserved portions of the variable region are called framework regions (FRs). The variable regions of the natural heavy and light chains respectively contain four FR regions, which generally have a β-sheet configuration and are connected by three CDRs that form a connecting loop, forming partial β-sheet structures in some cases. The CDRs in each chain are closely clustered together through the FR regions and together with the CDRs of the other chain, forming the antigen-binding site of the antibody (see Kabat et al., NIH Publ. No. 91-3242, Vol. I, pages 647-669 (1991)). The constant regions do not directly participate in the binding of the antibody to the antigen, but they exhibit different effector functions, such as participating in antibody-dependent cytotoxicity of the antibody.

The antibodies of the present application can include, but are not limited to, polyclonal, monoclonal, monospecific, multispecific, bispecific, human, humanized, primateized, chimeric and single-chain antibodies. The antibodies disclosed herein can be from any animal origin, including birds and mammals. Preferably, the antibodies are human, mouse, donkey, rabbit, goat, guinea pig, camel, llama, horse or chicken antibodies.

The term "antibody fragment" or "antigen binding fragment" refers to a portion of an antibody, such as F(ab')2, F(ab)2, Fab', Fab, Fv, single-chain Fvs (scFv), single-chain antibodies, disulfide-linked Fvs (sdFv), fragments containing VL or VH domains, fragments produced by Fab expression libraries, and anti-idiotypic (anti-Id) antibodies. Regardless of structure, antibody fragments bind to the same antigen as the intact antibody recognizes. The term "antibody fragment" includes DARTs and diabodies. The term "antibody fragment" also includes any synthetic or genetically engineered protein that comprises an immunoglobulin variable region and acts like an antibody by forming a complex with a specific antigen. A "single-chain variable fragment" or "scFv" is a fusion protein of the variable regions of the heavy chain (VH) and light chain (VL) of an immunoglobulin. In some embodiments, the domain regions are connected by a short linker peptide of 10 to about 25 amino acids. The linker can be glycine-rich for flexibility and serine-rich or threonine-rich for solubility, and can be linked to the N-terminus of VH or the C-terminus of VL, or vice versa. Despite the removal of the constant region and the introduction of the linker, this protein still retains the specificity of the original immunoglobulin. For IgG, standard immunoglobulin molecules contain two identical light chain polypeptides of approximately 23,000 daltons and two identical heavy chain polypeptides of 53,000-70,000 daltons. The four chains are typically linked by disulfide bonds in a "Y" configuration, with the light chains connecting (bracketing) the heavy chains from the mouth of the "Y" and extending through the variable region.

As described above, the variable region allows the antibody to selectively recognize and specifically bind to an epitope on the antigen. That is, the VL domain and VH domain of the antibody or a subset of the complementarity determining regions (CDRs) of the antibody combine to form the variable region that defines the three-dimensional antigen binding site. This tetrameric antibody structure forms the antigen binding site present at the end of each arm of the Y configuration. More specifically, the antigen binding site is defined by the three CDRs (i.e., HCDR1, HCDR2, HCDR3, LCDR1, LCDR2, and LCDR3) on each of the VH and VL chains. In some cases, for example, certain immunoglobulin molecules are derived from camelid animal species or engineered based on camelid immunoglobulin. Alternatively, immunoglobulin molecules may be composed of only heavy chains without light chains or only light chains without heavy chains.

In naturally occurring antibodies, the six CDRs present in each antigen binding domain are short, non-contiguous amino acid sequences that are specifically positioned to form the "antigen binding domain" due to the three-dimensional configuration of the antibody in an aqueous environment. The remaining amino acids in the antigen binding domain, called "framework" region domains, exhibit less intermolecular variability. The framework regions adopt a predominantly β-sheet conformation, and the CDRs form loops that are connected and form a part of the β-sheet structure in some cases. Thus, the framework region acts as a scaffold that positions the CDRs in the correct orientation through interchain non-covalent interactions. The antigen binding domain formed by the positioned CDRs defines a surface that is complementary to the epitope on the immunoreactive antigen. This complementary surface promotes non-covalent binding of the antibody to its cognate epitope. Since they have been precisely defined, the ordinary skilled person in the art of domain structures can readily identify the amino acids comprising the CDRs and framework regions, respectively, for any given heavy or light chain variable region.

As used herein, the term "light chain constant region (CL)" encompasses the amino acid sequence CL (SEQ ID NO: 30 or 31) derived from an antibody light chain. Preferably, the light chain constant region comprises at least one of the constant kappa domain or the constant lambda domain.

As used herein, the term "heavy chain constant region (CH)" encompasses the amino acid sequence derived from an immunoglobulin heavy chain. A polypeptide comprising a heavy chain constant region comprises at least one of the following: a CH1 domain (SEQ ID NO: 26 or 28), a hinge region (e.g., an upper, middle, and/or lower hinge region) domain, a CH2 domain, a CH3 domain, or a variant or fragment thereof. It is understood that the heavy chain constant regions can be modified such that their amino acid sequences differ from those of naturally occurring immunoglobulin molecules.

As used herein, a "variant" of an antibody, antibody fragment, or antibody domain refers to an antibody, antibody fragment, or antibody domain that: (1) has at least 80%, 85%, 90%, 95%, 96%, 97%, 98%, or 99% sequence identity to the original antibody, antibody fragment, or antibody domain, and (2) specifically binds to the same target as the original antibody, antibody fragment, or antibody domain specifically binds. It should be understood that in the case of the sequence identity being expressed in the form of "at least x% identical" or "at least x% identity", such embodiments include any and all numerical percentages equal to or greater than the lower limit. Further, it should be understood that in the presence of amino acid sequences in the present application, they should be construed as additionally disclosing or encompassing those having at least 80%, at least 85%, at least 90%, at least 95%, at least 96%, at least 97%, at least 98%, or at least 99% identity to said amino acid sequence.

The scope of the multi-specific molecules of the present invention includes a variety of compositions and methods, including: asymmetric IgG-like antibodies (e.g., trifunctional monoclonal antibodies (triomabs)/quadromas); knobs-into-holes antibodies; Cross monoclonal antibodies (Cross MAb); electrostatically matched antibodies; LUZ-Y; chain-exchange engineered domain (SEED) bodies; Fab-exchanged antibodies, symmetric IgG class antibodies; two-in-one antibodies; crosslinked monoclonal antibodies, mAb2; Cov X-body; dual variable domain (DVD)-Ig fusion proteins; IgG-like bispecific antibodies; Ts2Ab; BsAb; scFv/Fc fusions; dual (scFv)2-Fabs; F(ab)2 fusion proteins; bifunctional or Bis-Fab; Dock-and-Lock(DNL); Fab-Fv; scFv-based antibodies and bispecific antibody-based antibodies, such as bispecific T cell engagers (BiTEs); tandem bispecific antibodies (TandAb); DARTs; single-chain bispecific antibodies; TCR-like antibodies; human serum albumin scFv fusion proteins, COMBODIES and IgG/non-IgG fusion proteins.

As used herein, the phrase "multi-specific T cell engager" refers to a molecule comprising at least two targeting domains having different binding specificities, wherein at least one targeting domain specifically binds to a T cell surface antigen. In some embodiments, the multi-specific inhibitor is a polypeptide comprising a scaffold and two or more immunoglobulin antigen binding domains targeting different antigens or epitopes. In some embodiments, the multi-specific T cell engager is a bispecific antibody. In some other embodiments, the multi-specific T cell engager is a trispecific antibody.

As used herein, the phrase "bispecific" refers to a molecule comprising at least two targeting domains having different binding specificities. Each targeting domain is capable of specifically binding to a target molecule and, upon binding to the target molecule, inhibits the biological function of the target molecule. In some embodiments, the bispecific T cell engager is a polymeric molecule having two or more peptides. In some embodiments, the targeting domain comprises the antigen binding domain or CDRs of an antibody. In some embodiments, the targeting domain comprises a ligand or fragment thereof that specifically binds to a target protein. In some embodiments, the bispecific inhibitor is a bispecific antibody.

The terms "bispecific antibody" and "bispecific T cell engager" can be used interchangeably herein to refer to an antibody that can specifically bind to two different antigens (or epitopes). In some embodiments, the bispecific antibody is a full-length antibody that binds one antigen (or epitope) on one of its two binding arms (one pair of HC/LC) and binds to a different antigen (or epitope) on its second arm (the other pair of HC/LC). In these embodiments, the bispecific antibody has two different antigen binding arms (both in terms of specificity and CDR sequence) and is monovalent for each antigen it binds to.

In some other embodiments, the bispecific antibody is a full-length antibody that can bind two different antigens (or epitopes) in each of its two binding arms (two pairs of HC/LC). In these embodiments, the bispecific antibody has two identical antigen binding arms, with the same specificity and the same CDR sequence, and is bivalent for each antigen it binds to.

The terms "trispecific antibody" and "trispecific T cell engager" can be used interchangeably herein to refer to a molecule comprising three targeting domains having three different binding specificities. Each targeting domain is capable of specifically binding to a target molecule and, upon binding to the target molecule, inhibits the biological function of the target molecule. In some embodiments, the trispecific antagonist is a polymeric molecule having two or more peptides. In some embodiments, the targeting domain comprises the antigen binding domain or CDRs of an antibody. In some embodiments, the targeting domain comprises a ligand or fragment thereof that specifically binds to a target protein.

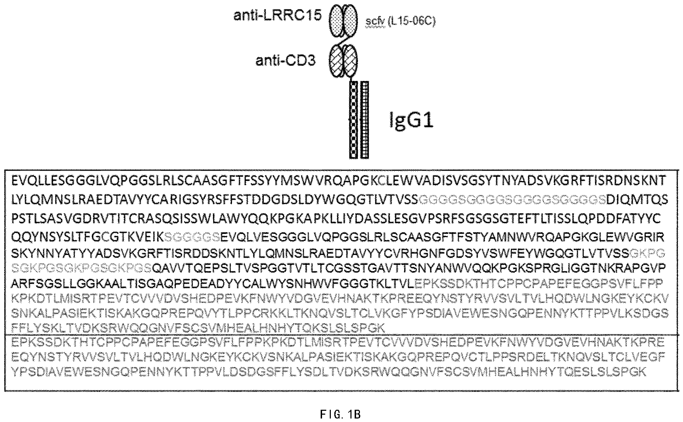

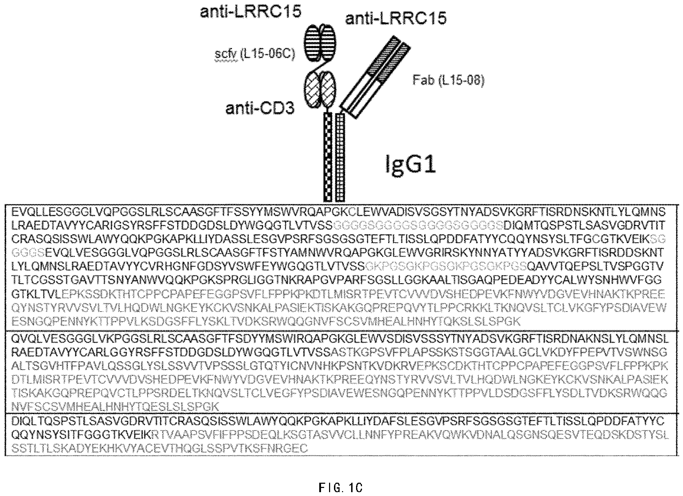

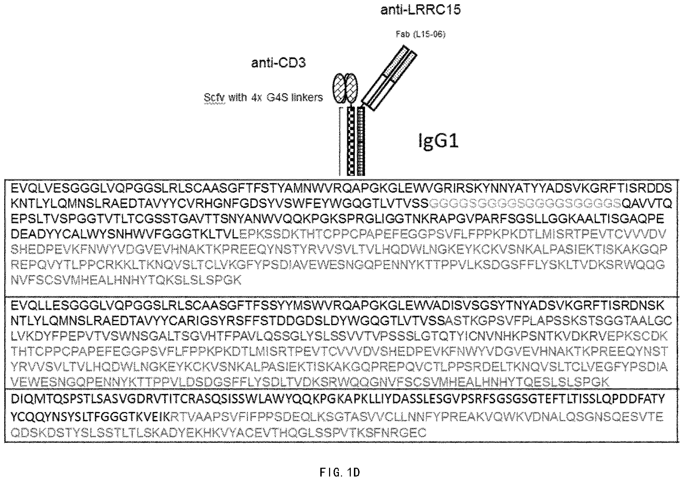

In a preferred embodiment of the present invention, a bispecific antibody targeting LRRC15 and CD3 is constructed, having the structure shown in Figure 1A a-c. An exemplary molecule of structure a is shown in Figure 1D, wherein the anti-CD3 scFv can be an scFv constructed from the VH and VL of any anti-CD3 antibody described in the present invention; the anti-LRRC15 Fab can be a Fab constructed from the VH and VL of any anti-LRRC15 antibody described in the present invention. An exemplary molecule of structure b is shown in Figure 1B, wherein the anti-LRRC15 scFv can be an scFv constructed from the VH and VL of any anti-LRRC15 antibody described in the present invention; the anti-CD3 scFv can be an scFv constructed from the VH and VL of any anti-CD3 antibody described in the present invention. An exemplary molecule of structure c is shown in Figure 1C, wherein the anti-LRRC15 scFv can be an scFv constructed from the VH and VL of any anti-LRRC15 antibody described in the present invention; the anti-CD3 scFv can be an scFv constructed from the VH and VL of any anti-CD3 antibody described in the present invention; the anti-LRRC15 Fab can be a Fab constructed from the VH and VL of any anti-LRRC15 antibody described in the present invention.

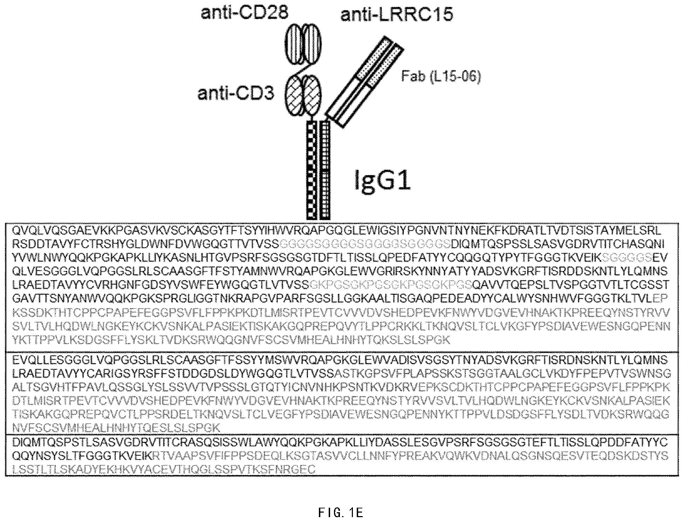

In another preferred embodiment of the present invention, a bispecific antibody targeting LRRC15, CD3 and CD28 is constructed, having the structure shown in Figure 1A d-e. An exemplary molecule of structure e is shown in Figure 1E, wherein the anti-CD28 scFv can be an scFv constructed from the VH and VL of any anti-CD28 antibody described in the present invention; the anti-CD3 scFv can be an scFv constructed from the VH and VL of any anti-CD3 antibody described in the present invention; the anti-LRRC15 Fab can be a Fab constructed from the VH and VL of any anti-LRRC15 antibody described in the present invention.

In another preferred embodiment of the present invention, a bispecific antibody targeting LRRC15 and CD40 is constructed, having the structure shown in Figure 2A a-d. An exemplary molecule of structure a is shown in Figure 2C, wherein the anti-LRRC15 Fab can be a Fab constructed from the VH and VL of any anti-LRRC15 antibody described in the present invention; CD40L can be any CD40L described in the present invention. An exemplary molecule of structure b is shown in Figure 2D, wherein the anti-LRRC15 Fab can be a Fab constructed from the VH and VL of any anti-LRRC15 antibody described in the present invention; CD40L can be any CD40L described in the present invention. An exemplary molecule of structure c is shown in Figure 2B, wherein the anti-LRRC15 Fab can be a Fab constructed from the VH and VL of any anti-LRRC15 antibody described in the present invention; the anti-CD40 scFv can be an scFv constructed from the VH and VL of any anti-CD40 antibody described in the present invention. An exemplary molecule of structure d is shown in Figure 2E, wherein the anti-CD40 Fab can be a Fab constructed from the VH and VL of any anti-CD40 antibody described in the present invention; the anti-LRRC15 scFv can be an scFv constructed from the VH and VL of any anti-LRRC15 antibody described in the present invention.

In another preferred embodiment of the present invention, a bispecific antibody targeting LRRC15 and CD137 is constructed, having the structure shown in Figure 3A a-d. An exemplary molecule of structure a is shown in Figure 3C, wherein the anti-LRRC15 Fab can be a Fab constructed from the VH and VL of any anti-LRRC15 antibody described in the present invention; CD137L can be any CD137L described in the present invention. An exemplary molecule of structure b is shown in Figure 3D, wherein the anti-LRRC15 Fab can be a Fab constructed from the VH and VL of any anti-LRRC15 antibody described in the present invention; CD137L can be any CD137L described in the present invention. An exemplary molecule of structure c is shown in Figure 3B, wherein the anti-LRRC15 Fab can be a Fab constructed from the VH and VL of any anti-LRRC15 antibody described in the present invention; the anti-CD137 scFv can be an scFv constructed from the VH and VL of any anti-CD137 antibody described in the present invention. An exemplary molecule of structure d is shown in Figure 3E, wherein the anti-CD137 Fab can be a Fab constructed from the VH and VL of any anti-CD137 antibody described in the present invention; the anti-LRRC15 scFv can be an scFv constructed from the VH and VL of any anti-LRRC15 antibody described in the present invention.

The present invention also provides polynucleotide molecules encoding the aforementioned antibodies or fragments thereof. The polynucleotides of the present invention can be in DNA or RNA form. DNA forms include cDNA, genomic DNA, or synthetic DNA. DNA can be single-stranded or double-stranded. DNA can be the coding strand or the non-coding strand. The coding region sequence encoding the mature polypeptide can be the same as or a degenerate variant of the coding region sequence of the antibody of the present invention. As used herein, "degenerate variant" in the present invention refers to a nucleic acid sequence that encodes a polypeptide having the same amino acid sequence as the polypeptide of the present invention, but has a different coding region sequence.

Polynucleotides encoding the mature polypeptides of the present invention include: coding sequences encoding only the mature polypeptide; coding sequences for the mature polypeptide and various additional coding sequences; coding sequences for the mature polypeptide (and optionally additional coding sequences) and non-coding sequences.

The term "polynucleotide encoding a polypeptide" can be a polynucleotide that includes the coding sequence for this polypeptide, or it can be a polynucleotide that futher includes additional coding and/or non-coding sequences.

The present invention also relates to polynucleotides that hybridize to the above sequences and have at least 50%, preferably at least 70%, more preferably at least 80% identity between the two sequences. The present invention particularly relates to polynucleotides that can hybridize to the polynucleotides described in the present invention under stringent conditions. In the present invention, "stringent conditions" refers to: (1) hybridization and washing at low ionic strength and high temperature, such as 0.2×SSC, 0.1%SDS, 60°C; or (2) hybridization with a denaturant, such as 50% (v/v) formamide, 0.1% calf serum/0. 1% Ficoll, 42°C, etc.; or (3) hybridization only occurs when the identity between the two sequences is at least 90%, preferably above 95%. Moreover, the polypeptides encoded by the polynucleotides that can hybridize have the same biological function and activity as the mature polypeptides shown in SEQ ID NO.4 and SEQ ID NO.9.

The full-length nucleotide sequence of the antibody of the present invention or a fragment thereof can generally be obtained by PCR amplification, recombination or artificial synthesis. A feasible method is to use artificial synthesis to synthesize the related sequences, especially when the fragment length is shorter. Usually, a very long fragment can be obtained by first synthesizing multiple small fragments and then ligating them. In addition, the coding sequence of the heavy chain can be fused with an expression tag (such as 6His) to form a fusion protein.

Once the relevant sequence is obtained, the relevant sequence can be obtained in large quantities by recombination. This is usually done by cloning it into a vector, transforming it into a cell, and then isolating the relevant sequence from the proliferated host cells by conventional methods. The biological molecules (nucleic acids, proteins, etc.) involved in the present invention include biological molecules that exist in an isolated form.

At present, it is possible to obtain the DNA sequence encoding the protein of the present invention (or its fragment, or its derivative) completely by chemical synthesis. The DNA sequence can then be introduced into various existing DNA molecules (or vectors) and cells known in the art. In addition, mutations can be introduced into the protein sequence of the present invention by chemical synthesis.

The present invention also relates to vectors comprising the appropriate DNA sequences described above and appropriate promoter or control sequences. These vectors can be used to transform appropriate host cells to enable them to express proteins.

The host cell can be a prokaryotic cell, such as a bacterial cell; or a lower eukaryotic cell, such as a yeast cell; or a higher eukaryotic cell, such as a mammalian cell. Representative examples include: *Escherichia coli, Streptomyces*; bacterial cells of *Salmonella typhimurium*; fungal cells such as yeast; insect cells of Drosophila S2 or Sf9; animal cells of CHO, COS7, 293 cells, etc.

Transformation of host cells with recombinant DNA can be carried out using conventional techniques well known to those skilled in the art. When the host is a prokaryotic organism such as *Escherichia coli,* competent cells capable of taking up DNA can be harvested after the exponential growth phase and treated with the CaCl₂ method, the steps of which are well known in the art. Another method is to use MgCl₂. If necessary, transformation can also be carried out by electroporation. When the host is a eukaryote, the following DNA transfection methods can be selected: calcium phosphate coprecipitation method, conventional mechanical methods such as microinjection, electroporation, liposome packaging, etc.

The obtained transformants can be cultured by conventional methods to express the polypeptide encoded by the gene of the present invention. Depending on the host cell used, the culture medium used in the culture can be selected from various conventional culture media. The culture is carried out under conditions suitable for the growth of the host cell. When the host cells grow to an appropriate cell density, the selected promoter is induced by an appropriate method (such as temperature shift or chemical induction), and the cells are cultured for a further period of time.

In the above methods, the recombinant polypeptide can be expressed intracellularly, or on the cell membrane, or secreted extracellularly. If necessary, the recombinant protein can be separated and purified by various separation methods using its physical, chemical and other properties. These methods are well known to those skilled in the art. Examples of these methods include but are not limited to: conventional renaturation treatment, treatment with protein precipitants (salting out method), centrifugation, osmotic lysis, ultrafiltration, ultracentrifugation, molecular sieve chromatography (gel filtration), adsorption chromatography, ion exchange chromatography, high performance liquid chromatography (HPLC) and other various liquid chromatography techniques and combinations of these methods.

The antibodies of the present invention can be used alone or in combination or conjugated with detectable labels (for diagnostic purposes), therapeutic agents, PK (protein kinase) modification moieties, or any combination of the above substances.

Detectable labels for diagnostic purposes include but are not limited to: fluorescent or luminescent labels, radioactive labels, MRI (magnetic resonance imaging) or CT (computed tomography) contrast agents, or enzymes capable of producing detectable products.

Couplable therapeutic agents include but are not limited to: insulin, IL-2, interferon, calcitonin, GHRH peptide, enterostatin analogs, albumin, antibody fragments, cytokines, and hormones.

The present invention also provides a composition. In a preferred embodiment, the composition is a pharmaceutical composition, which comprises the aforementioned antibody or active fragment or fusion protein thereof, and a pharmaceutically acceptable carrier. Typically, these substances can be formulated in a non-toxic, inert and pharmaceutically acceptable aqueous carrier medium, the pH of which is usually about 5-8, preferably about 6-8, although the pH value may vary depending on the nature of the substance being formulated and the disease to be treated. The prepared pharmaceutical composition can be administered by conventional routes, including but not limited to: oral, respiratory, intra-tumoral, intraperitoneal, intravenous, or topical administration.

The pharmaceutical composition of the present invention can be used for the treatment of cancer/tumors, especially solid tumors, especially solid tumors with high LCRR15 expression.

The pharmaceutical composition of the present invention comprises a safe and effective amount (such as 0.001-99wt%, preferably 0.01-90wt%, more preferably 0.1-80wt%) of the aforementioned monoclonal antibody (or conjugate thereof) of the present invention and a pharmaceutically acceptable carrier or excipient. Such carriers include but are not limited to: saline, buffers, glucose, water, glycerol, ethanol, and a combination thereof. The pharmaceutical formulation should be compatible with the route of administration. The pharmaceutical composition of the present invention can be prepared in the form of an injection, for example, by conventional methods using a physiological saline solution or an aqueous solution containing glucose and other excipients. The pharmaceutical composition, such as an injection or solution, should be manufactured under aseptic conditions. The dosage of the active ingredient is a therapeutically effective amount, for example about 1 microgram/kg body weight to about 10 milligrams/kg body weight per day. In addition, the pharmaceutical composition of the present invention can also be used together with other therapeutic agents.

When using the pharmaceutical composition, a safe and effective amount of the immunoconjugate is administered to a mammal, wherein the safe and effective amount is usually at least about 10 micrograms/kg body weight and in most cases does not exceed about 8 milligrams/kg body weight, preferably the dose is about 10 micrograms/kg body weight to about 1 milligram/kg body weight. Of course, the specific dosage should also consider the route of administration, the patient's health status, etc., which are all within the scope of the skills of a skilled physician.

### The main advantages of the present invention include:

(1) Cancer-associated fibroblasts (CAFs) are an important component of the micro environment of most solid tumors. These cells promote cancer progression by supporting the growth of tumor cells, participating in the remodeling of the tumor extracellular matrix, promoting angiogenesis, and regulating tumor-induced inflammation. CAFs coexist with other cells such as endothelial cells and immune cells expressing CD40 in the cancer stroma. Thus, since LRRC15 is specifically expressed on CAFs, bispecific/trispecific antibodies can be designed for this target to bring immune activation signals into the tumor stroma, thereby helping to the transform of cold tumors into hot tumors in the treatment of most solid tumors.
(2) Compared with ordinary tumor-associated antigen (TAA) targets, the expression of LRRC15 on multiple solid tumors and CAFs makes the bispecific/trispecific antibodies containing the LRRC15 antigen binding domain in the present invention more widely used.
(3) Due to the barrier effect of the tumor stroma, it is difficult for ordinary TAA targeting bispecific antibodies to directly bring immune regulators to the location of tumor cells, while bispecific antibodies that target LRRC15 expressed on CAFs at one end can easily induce immune cell activation by binding and activating regulatory targets (such as targeting TNFRSF) at the other end, thereby playing a role in the tumor stroma.
(4) The LRRC15-related bispecific/trispecific antibodies of the present invention can effectively activate T cells and reduce the matrix effects of tumors.

The present invention is further explained below in conjunction with specific examples. It should be understood that these examples are only for illustration of the present invention and not for limiting the scope of the present invention. The experimental methods not specifically mentioned in the following examples are generally carried out under conventional conditions, such as those described in Sambrook et al., Molecular Cloning: A Laboratory Manual (New York: Cold Spring Harbor Laboratory Press, 1989), or according to the conditions recommended by the manufacturer. Unless otherwise specified, percentages and parts are percentages by weight and parts by weight.

### Sequences used to prepare the multi-specific T cell engager of the present invention

### Sequences of LRRC15 antibodies

| | HCDR1 | HCDR2 | HCDR3 |
|---|---|---|---|
| L15-06H/ L15-06C H | GFTFSSYYMS (SEQ ID NO: 32) | DISVSGSYTNYADSV KG (SEQ ID NO: 33) | IGSYRSFFSTDDGDSL DY (SEQ ID NO: 34) |
| L15-08H | GFTFSDYYMS (SEQ ID NO: 35) | DISVSSSYTNYADSV KG (SEQ ID NO: 36) | LGGYRSFFSTDDGDSL DY (SEQ ID NO: 37) |
| L15-03H | GGTFSSYTIS (SEQ ID NO: 38) | GIIPMFGTANYAQKF QG (SEQ ID NO: 39) | GVALLATDFDY (SEQ ID NO: 40) |
| L15-07H | GGTFSTYAIS (SEQ ID NO: 41) | GITPMFGTPNYAQKF QG (SEQ ID NO: 42) | GDWYDYHFDY (SEQ ID NO: 43) |

| | LCDR1 | LCDR2 | LCDR3 |
|---|---|---|---|
| L15-06L/ L15-06C L | RASQSISSWLA (SEQ ID NO: 44) | DASSLES (SEQ ID NO: 45) | QQYNSYSLT (SEQ ID NO: 46) |
| L15-08L | RASQSISSWLA (SEQ ID NO: 44) | DAFSLES (SEQ ID NO: 47) | QQYNSYSIT (SEQ ID NO: 48) |
| L15-03L | TGTSSDVGGYNY VS (SEQ ID NO: 49) | EVSNRPS (SEQ ID NO: 50) | QSWTHDNSVV (SEQ ID NO: 51) |
| L15-07L | TGTSSDVGGYNY VS (SEQ ID NO: 49) | EVSNRPS (SEQ ID NO: 50) | QSYANRDNFVV (SEQ ID NO: 52) |

| | |
|---|---|
| EVQLLESGGGLVQPGGSLRLSCAASGFTFSSYYMSWVRQAPG KGLEWVADISVSGSYTNYADSVKGRFTISRDNSKNTLYLQMN SLRAEDTAVYYCARIGSYRSFFSTDDGDSLDYWGQGTLVTVS S | VH; L15-06H (SEQ ID NO: 1) |
| QVQLVESGGGLVKPGGSLRLSCAASGFTFSDYYMSWIRQAPG KGLEWVSDISVSSSYTNYADSVKGRFTISRDNAKNSLYLQMN SLRAEDTAVYYCARLGGYRSFFSTDDGDSLDYWGQGTLVTVS S | VH; L15-08H (SEQ ID NO: 2) |
| QVQLVQSGAEVKKPGSSVKVSCKASGGTFSSYTISWVRQAPG QGLEWMGGIIPMFGTANYAQKFQGRVTITADESTSTAYMELS SLRSEDTAVYYCARGVALLATDFDYWGQGTLVTVSS | VH; L15-03H (SEQ ID NO: 3) |
| QVQLVQSGAEVKKPGSSVKVSCKASGGTFSTYAISWVRQAPG QGLEWMGGITPMFGTPNYAQKFQGRVTITADESTSTAYMELS SLRSEDTAVYYCARGDWYDYHFDYWGQGTLVTVSS | VH; L15-07H (SEQ ID NO: 4) |
| DIQMTQSPSTLSASVGDRVTITCRASQSISSWLAWYQQKPGKA PKLLIYDASSLESGVPSRFSGSGSGTEFTLTISSLQPDDFATYYC QQYNSYSLTFGGGTKVEIK | VL; L15-06L (SEQ ID NO: 5) |
| DIQLTQSPSTLSASVGDRVTITCRASQSISSWLAWYQQKPGKA PKLLIYDAFSLESGVPSRFSGSGSGTEFTLTISSLQPDDFATYYC QQYNSYSITFGGGTKVEIK | VL; L15-08L (SEQ ID NO: 6) |
| QSALTQPASVSGSPGQSITISCTGTSSDVGGYNYVSWYQQHPG KAPKLMIYEVSNRPSGVSNRFSGSKSGNTASLTISGLQAEDEA DYYCQSWTHDNSVVFGGGTKLTVL | VL; L15-03L (SEQ ID NO: 7) |
| QSALTQPASVSGSPGQSITISCTGTSSDVGGYNYVSWYQQHPG KAPKLMIYEVSNRPSGVSNRFSGSKSGNTASLTISGLQAEDEA DYYCQSYANRDNFVVFGGGTKLTVL | VL; L15-07L (SEQ ID NO: 8) |
| EVQLLESGGGLVQPGGSLRLSCAASGFTFSSYYMSWVRQAPG KCLEWVADISVSGSYTNYADSVKGRFTISRDNSKNTLYLQMN SLRAEDTAVYYCARIGSYRSFFSTDDGDSLDYWGQGTLVTVS S | VH; L15-06CH (SEQ ID NO: 53) |
| DIQMTQSPSTLSASVGDRVTITCRASQSISSWLAWYQQKPGKA PKLLIYDASSLESGVPSRFSGSGSGTEFTLTISSLQPDDFATYYC QQYNSYSLTFGCGTKVEIK | VL; L15-06CL (SEQ ID NO: 54) |

### Sequences of CD3 antibodies

| | |
|---|---|
| EVQLVESGGGLVQPGGSLRLSCAASGFTFSTYAMNWVRQAPG KGLEWVGRIRSKANNYATYYADSVKGRFTISRDDSKNTLYLQ MNSLRAEDTAVYYCVRHGNFGDSYVSWFAYWGQGTLVTVSS | VH; CD3vB (SEQ ID NO: 9) |
| EVQLVESGGGLVQPGGSLRLSCAASGFTFSTYAMNWVRQAPG KGLEWVGRIRSKSNNYATYYADSVKGRFTISRDDSKNTLYLQ MNSLRAEDTAVYYCVRHGNFGDSYVSWFAYWGQGTLVTVSS | VH; CD3vG (SEQ ID NO: 10) |
| EVQLVESGGGLVQPGGSLRLSCAASGFTFSTYAMNWVRQAPG KGLEWVGRIRSKYNNYATYYADSVKGRFTISRDDSKNTLYLQ MNSLRAEDTAVYYCVRHGNFGDSYVSWFEYWGQGTLVTVSS | VH; CD3vI (SEQ ID NO: 11) |
| EVQLVESGGGLVQPGGSLRLSCAASGFTFSTYAMNWVRQAPG KGLEWVGRIRSKYNNYATYYADSVKGRFTISRDDSKNTLYLQ MNSLRAEDTAVYYCVRHGNFGDSYVSWFDYWGQGTLVTVSS | VH; CD3vJ (SEQ ID NO: 12) |
| QAVVTQEPSLTVSPGGTVTLTCGSSTGAVTTSNYANWVQQKP GKSPRGLIGGTNKRAPGVPARFSGSLLGGKAALTISGAQPEDE ADYYCALWYSNHWVFGGGTKLTVL | VL; CD3 (SEQ ID NO: 13) |

### Sequences of CD28 antibody

| | |
|---|---|
| QVQLVQSGAEVKKPGASVKVSCKASGYTFTSYYIHWVRQAP GQGLEWIGSIYPGNVNTNYNEKFKDRATLTVDTSISTAYMELS RLRSDDTAVYFCTRSHYGLDWNFDVWGQGTTVTVSS | VH; CD28 (C50S) (SEQ ID NO: 14) |
| DIQMTQSPSSLSASVGDRVTITCHASQNIYVWLNWYQQKPGK APKLLIYKASNLHTGVPSRFSGSGSGTDFTLTISSLQPEDFATY YCQQGQTYPYTFGGGTKVEIK | VL; CD28 (SEQ ID NO: 15) |

### Sequences of CD40 antibodies

| | |
|---|---|
| QVQLVQSGAEVKKPGASVKVSCKASGYTFTGYYMHWVRQA PGQGLEWMGWINPDSGGTNYAQKFQGRVTMTRDTSISTAYM ELNRLRSDDTAVYYCARDQPLGYCTNGVCSYFDYWGQGTLV TVSS | VH; CD40 (SEQ ID NO: 16) |
| DIQMTQSPSSVSASVGDRVTITCRASQGIYSWLAWYQQKPGK APNLLIYTASTLQSGVPSRFSGSGSGTDFTLTISSLQPEDFATY YCQQANIFPLTFGGGTKVEIK | VL; CD40 (SEQ ID NO: 17) |
| | VH; CD40d (SEQ ID NO: 55) |
| | VL; CD40d (SEQ ID NO: 56) |

### Sequences of CD137 antibodies

| | |
|---|---|
| | VH; CD137e (SEQ ID NO: 18) |
| | VL; CD137e (SEQ ID NO: 19) |
| EVQLVQSGAEVKKPGESLRISCKGSGYSFSTYWISWVRQMPG KGLEWMGKIYPGDSYTNYSPSFQGQVTISADKSISTAYLQWSS LKASDTAMYYCARGYGIFDYWGQGTLVTVSS | VH; CD137o (SEQ ID NO: 20) |
| SYELTQPPSVSVSPGQTASITCSGDNIGDQYAHWYQQKPGQSP VLVIYQDKNRPSGIPERFSGSNSGNTATLTISGTQAMDEADYY CATYTGFGSLAVFGGGTKLTVL | VL; CD137o (SEQ ID NO: 21) |

### Sequences of CD40 ligand (CD40L)

Variants of CD40LRBD can use one of the following segments: 112-261 segment, 113-261 segment, 116-261 segment, 117-261 segment, 119-261 segment, and 121-261 segment in the CD40 ligand sequence.

| | |
|---|---|
| HRRLDKIEDERNLHEDFVFMKTIQRCNTGERSLSLLNCEEIKS QFEGFVKDIMLNKEETKKENSFEMQKGDQNPQIAAHVISEASS KTTSVLQWAEKGYYTMSNNLVTLENGKQLTVKRQGLYYIYA QVTFCSNREASSQAPFIASLCLKSPGRFERILLRAANTHSSAKP CGQQSIHLGGVFELQPGASVFVNVTDPSQVSHGTGFTSFGLLK L | CD40LECD (47-261) (SEQ ID NO: 22) |
| NPQIAAHVISEASSKTTSVLQWAEKGYYTMSNNLVTLENGKQ LTVKRQGLYYIYAQVTFCSNREASSQAPFIASLCLKSPGRFERI LLRAANTHSSAKPCGQQSIHLGGVFELQPGASVFVNVTDPSQ VSHGTGFTSFGLLKL | CD40LRBD (119-261) (SEQ ID NO: 23) |

### Sequences of CD137 ligand (CD137L)

Variants of CD137LRBD can use one of the following segments: 52-248 segment, 58-248 segment, 71-248 segment, 80-248 segment, 85-248 segment, 52-254 segment, 58-254 segment, 71-254 segment, 80-254 segment, and 85-254 segment in the CD137 ligand sequence.

| | |
|---|---|
| ACPWAVSGARASPGSAASPRLREGPELSPDDPAGLLDLRQGM FAQLVAQNVLLIDGPLSWYSDPGLAGVSLTGGLSYKEDTKEL VVAKAGVYYVFFQLELRRVVAGEGSGSVSLALHLQPLRSAA GAAALALTVDLPPASSEARNSAFGFQGRLLHLSAGQRLGVHL HTEARARHAWQLTQGATVLGLFRVTPEIPAGLPSPRSE | CD137LEC D (50-254) (SEQ ID NO: 24) |
| REGPELSPDDPAGLLDLRQGMFAQLVAQNVLLIDGPLSWYSD PGLAGVSLTGGLSYKEDTKELVVAKAGVYYVFFQLELRRVV AGEGSGSVSLALHLQPLRSAAGAAALALTVDLPPASSEARNS AFGFQGRLLHLSAGQRLGVHLHTEARARHAWQLTQGATVLG LFRVTPEIPAGLPSPRSE | CD 13 7LRB D (71-254) (SEQ ID NO: 25) |

### Sequence of IgG1 CH

| | |
|---|---|
| ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKRVEPKSCDKTHTCPPCPAPELLGGPSVFLFP PKPKDTLMISRTPEVTCVVVDVSHEDPEVKFNWYVDGVEV HNAKTKPREEQYNSTYRVVSVLTVLHQDWLNGKEYKCKV SNKALPAPIEKTISKAKGQPREPQVYTLPPSRDELTKNQVS LTCLVKGFYPSDIAVEWESNGQPENNYKTTPPVLDSDGSFF LYSKLTVDKSRWQQGNVFSCSVMHEALHNHYTQKSLSLS PGK | CH1-3 IgG1 CH1 : (SEQ ID NO: 26) Fc: (SEQ ID NO: 27) |

The **bold** part is the Fc region.

The Fc region can contain the following mutations:

| Mutations to knock out FcyR function and/or the KIH or charged pairs mutations | Mutations to increase the Fcγ RIIb binidng |
|---|---|
| N297A, L234F/L235E/P331S, Y349C/K370E/K409D/K439E, S354C/D356K/E357K/D399K, S354C/T366W, Y349C/T366S/L368A/Y407V, L234F/L235E/P33lS/Y349C/K370E/K409D/K439E, L234F/L235E/P331S/S354C/D356K/E357K/D399K, L234F/L235E/P331S/S354C/T366W, L234F/L235E/P331S/Y349C/T366S/L368A/Y407V, N297A/Y349C/K370E/K409D/K439E, N297A/S354C/D356K/E357K/D399K, N297A/S354C/T366W, N297A/Y349C/T366S/L368A/Y407V. | S267E, S267E/G236D, S267E/S239D, S267E/L328F |

In a preferred embodiment of the present invention, the Fc region has the following sequences:

| | |
|---|---|
| EPKSSDKTHTCPPCPAPEFEGGPSVFLFPPKPKDTLMISRTP EVTCVVVDVSHEDPEVKFNWYVDGVEVHNAKTKPREEQ YNSTYRVVSVLTVLHQDWLNGKEYKCKVSNKALPASIEK TISKAKGQPREPQVYTLPPCRKKLTKNQVSLTCLVKGFYP SDIAVEWESNGQPENNYKTTPPVLKSDGSFFLYSKLTVDK SRWQQGNVFSCSVMHEALHNHYTQKSLSLSPGK | Fc(C220S/L234 F/L235E/P331S/ S354C/D356K/E 357K/D399K) (SEQ ID NO: 57) |
| EPKSCDKTHTCPPCPAPEFEGGPSVFLFPPKPKDTLMISRTP EVTCVVVDVSHEDPEVKFNWYVDGVEVHNAKTKPREEQ YNSTYRVVSVLTVLHQDWLNGKEYKCKVSNKALPASIEK TISKAKGQPREPQVCTLPPSRDELTKNQVSLTCLVEGFYPS DIAVEWESNGQPENNYKTTPPVLDSDGSFFLYSDLTVDKS RWQQGNVFSCSVMHEALHNHYTQESLSLSPGK | Fc(L234F/L235E /P331S/Y349C/ K370E/K409D/ K439E) (SEQ ID NO: 58) |
| EPKSCDKTHTCPPCPAPEFEGGPSVFLFPPKPKDTLMISRTP EVTCVVVDVEHEDPEVKFNWYVDGVEVHNAKTKPREEQ YNSTYRVVSVLTVLHQDWLNGKEYKCKVSNKALPASIEK | Fc(L234F/L235E /S267E/P331S) (SEQ ID NO: 59) |
| TISKAKGQPREPQVYTLPPSRDELTKNQVSLTCLVKGFYPS DIAVEWESNGQPENNYKTTPPVLDSDGSFFLYSKLTVDKS RWQQGNVFSCSVMHEALHNHYTQKSLSLSPGK | |
| EPKSCDKTHTCPPCPAPEFEGGPSVFLFPPKPKDTLMISRTP EVTCVVVDVSHEDPEVKFNWYVDGVEVHNAKTKPREEQ YNSTYRVVSVLTVLHQDWLNGKEYKCKVSNKALPASIEK TISKAKGQPREPQVYTLPPSRDELTKNQVSLTCLVKGFYPS DIAVEWESNGQPENNYKTTPPVLDSDGSFFLYSKLTVDKS RWQQGNVFSCSVMHEALHNHYTQKSLSLSPGK | Fc(L234F/L235E /P331S) (SEQ ID NO: 60) |

### Sequence of IgG4 CH

| | |
|---|---|
| ASTKGPSVFPLAPCSRSTSESTAALGCLVKDYFPEPVTVSWNS GALTSGVHTFPAVLQSSGLYSLSSVVTVPSSSLGTKTYTCNVD HKPSNTKVDKRVESKYGPPCPPCPAPEFLGGPSVFLFPPKP KDTLMISRTPEVTCVVVDVSQEDPEVQFNWYVDGVEVHN AKTKPREEQFNSTYRVVSVLTVLHQDWLNGKEYKCKVSN KGLPSSIEKTISKAKGQPREPQVYTLPPSQKKMTKNQVSLT CLVKGFYPSDIAVEWESNGQPENNYKTTPPVLKSDGSFFLY SRLTVDKSRWQEGNVFSCSVMHEALHNHYTQKSLSLSLG K | CH1-3 IgG4 (S228P) CH1 : (SEQ ID NO: 28) Fc: (SEQ ID NO: 29) |

The **bold** part is the Fc region.

The Fc region can contain the following mutations:

| Mutations of the KIH or the charged pairs | Mutations to increase the Fcγ RIIb binidng |
|---|---|
| Y349C/K370E/R409D/K439E, S354C/E356K/E357K/D399K, S354C/T366W, Y349C/T366S/L368A/Y407V | S228P/S267E, S228P/S267E/G236D, S228P/S267E/S239D, S228P/S267E/L328F |

### Sequences of CL

| | |
|---|---|
| | CL; Kappa (SEQ ID NO: 30) |
| GQPKAAPSVTLFPPSSEELQANKATLVCLISDFYPGAVTVAWK ADSSPVKAGVETTTPSKQSNNKYAASSYLSLTPEQWKSHRSY SCQVTHEGSTVEKTVAPTECS | CL; Lambda (SEQ ID NO: 31) |

### Optional linker sequences

| |
|---|
| G |
| GS |
| GSS |
| GSSSG |
| GGGGS |
| GGGGSGGGGS |
| GGGGSGGGGSGGGGS |
| GGGGSGGGGSGGGGSGGGGS |
| GGGGSGGGGSGGGGSGGGGSGGGGS |
| PGGGGSP |
| PGGGGSPGGGGSPGGGGSP |
| GEPGSGE |
| GEPGSGEGEPGSGE |
| GEPGSGEGEPGSGEEGEPGSGE |
| EGEPGSGEEGEPGSGEEGEPGSGEEGEPGSGE |
| GKPGS |
| GKPGSGKPGS |
| GKPGSGKPGSGKPGS |
| GKPGSGKPGSGKPGSGKPGS |
| GKPGSGKPGSGKPGSGKPGSGKPGS |
| SSSSG |
| SSSSGSSSSG |
| SSSSGSSSSGSSSSG |
| SSSSGSSSSGSSSSGSSSSG |
| GRPGSGPGSGRPGSGRPGS |
| GRPGSGPGSGRPGSGRPGSGRGPS |
| GKPGSGRPGSGKGPSGRPGS |

### Example 1

### Preparation and verification of anti-LRRC15 antibodies

The LRRC15 antibodies of the present invention were obtained by phage library screening. The phage screening technology is referenced in, for example, Kay, B. Winter, J. & McCafferty, J. (eds.) (1996) Phage Display of Polypeptides and Proteins: A Laboratory Manual (Academic Press). During the screening, specific binding molecules were screened for the extracellular domain of LRRC15 by ELISA and FACS methods, and the sequences were confirmed by sequencing. Finally, four candidate antibodies were obtained: L15-06, L15-08, L15-03 and L15-07.

Furthermore, the binding ability of the LRRC15 antibodies of the present invention to CHO cells expressing LRRC15 was tested. Therein, CHO cells expressing LRRC15 were prepared by the following method: the full-length sequence gene fragments of human, mouse and monkey LRRC15 were obtained by whole gene synthesis, and then these fragments were inserted into the backbone of the stable expression vector to obtain the expression plasmid vectors. These three plasmids were transfected into CHO cells, and monoclonal cell lines were screened under the pressure of puromycin, which are the flow cytometry cell lines used in this example.

CHO cells expressing LRRC15 were washed with FACS buffer (0.5% BSA in PBS) and resuspended at 2E6 cells/ml with the gradient diluted LRRC15 antibodies. The resuspension was incubated on ice for 30 minutes, then washed twice with FACS buffer and incubated with the second antibody on ice for 30 minutes. After washing the resuspended cells with FACS buffer again, the cells were detected by flow cytometry. Based on the measured mean fluorescence intensity, the half maximal binding concentration (EC50) of each antibody was determined.

The results of the analysis are shown in Figure 4A-C. The binding abilities of the four antibodies to cells expressing human LRRC15 were similar, but the binding abilities to cells expressing mouse LRRC15 were quite different. Among them, L15-06 had the strongest binding ability, followed by L15-08, L15-03 was weaker, and L15-07 was the weakest. In addition, all four antibodies have strong monkey cross-binding ability.

In addition, competitive experiments of antibody binding were also carried out: after the four monoclonal antibody molecules were bound to CHO cells expressing LRRC15, the biotin-labeled monoclonal antibody molecule was used to compete for binding, and then the signal intensity was detected by SA-FITC. The greater the decrease in signal, the stronger the competition. The results showed that L15-08 had almost no competitive effect on L15-L06, indicating that L15-06 and L15-08 bind to different epitopes of LRRC15 (Figure 4D).

### Example 2

### Preparation of multi-specific T cell engager molecules of the present invention

According to the four different LRRC15 antibody sequences obtained in Example 1 and the antibody sequence derived from L15-06 (L15-06C), a variety of multi-specific antibody molecules were designed.

The molecular sequence fragments of the multi-specific antibodies were obtained by the whole gene synthesis method, and then the target sequence was inserted into the expression vector by conventional gene cloning methods (see for example, Lo. B.K.C methods in Molecular Biology. Volume 248, 2004. Antibody Engineering).

HEK293E cells were transfected with the vector carrying the multi-specific molecule. The cells were cultured in F17 medium (1 L F17 + 10 mL 10% PF68 + 30 ml 200 mM L-glutamine) at 37°C, 5% CO₂, for 7 days, then the corresponding molecules can be produced. After the expression was finished, the supernatant was collected and purified to obtain the multi-specific T cell engager molecule, which could be used for various experimental analysis.

Using the above method, the following multi-specific molecules were prepared:
(1) Bispecific antibodies composed of LRRC15 antigen binding domain and CD3 antigen binding domain, as shown in structures a-c in Figure 1A; trispecific antibodies composed of LRRC15 antigen binding domain, CD3 antigen binding domain, and CD28 antigen binding domain, as shown in structures d-e in Figure 1A.
   A bispecific antibody of structure a in Figure 1A is named 315-L06, and its structure is shown in Figure 1D;
   A bispecific antibody of structure b is named 153-L06C, and its structure is shown in Figure 1B; another bispecific antibody of structure b is named 153-L07, which differs from 153-L06C in that the anti-LRRC15 scFv uses VH and VL of L15-07; another bispecific antibody of structure b is named 153-L06, which differs from 153-L06C in that the anti-LRRC15 scFv uses VH and VL of L15-06;
   A bispecific antibody of structure c is named 315-L06C08, and its structure is shown in Figure 1C; another bispecific antibody of structure c is named 315-L0608, which differs from 315-L06C08 in that the anti-LRRC15 scFv uses VH and VL of L15-06; another bispecific antibody of structure c is named 315-L0806, which differs from 315-L06C08 in that the anti-LRRC15 scFv uses VH and VL of L15-08, and the anti-LRRC15 Fab uses VH and VL of L15-06; another bispecific antibody of structure c is named 315-L08C06, which differs from 315-L06C08 in that the anti-LRRC15 scFv uses VH and VL of L15-08C, and the anti-LRRC15 Fab uses VH and VL of L15-06;

A trispecific antibody of structure e is named 28315-L06, and its structure is shown in Figure 1E.

(2) Bispecific antibodies composed of LRRC15 antigen binding domain and CD40 antigen binding domain, as shown in a-d of Figure 2A.
A bispecific antibody of structure a in Figure 2A is named 1540L-L06, and its structure is shown in Figure 2C;
A bispecific antibody of structure b is named 1540TL-L06, and its structure is shown in Figure 2D;
A bispecific antibody of structure c is named 1540-L06, and its structure is shown in Figure 2B;

A bispecific antibody of structure d is named 4015-L06, and its structure is shown in Figure 2E; another bispecific antibody of structure d is named 40D15-L06C, which differs from 4015-L06 in that the anti-CD40 IgG uses the VH and VL of CD40d, and the anti-LRRC15 scFv uses the VH and VL of L15-06C; another bispecific antibody of structure d is named 40D15-L06CS, which differs from 4015-L06 in that the anti-CD40 IgG uses the VH and VL of CD40d, the Fc fragment is replaced with the fragment shown in SEQ ID NO: 60, and the anti-LRRC15 scFv uses the VH and VL of L15-06C.

(3) Bispecific antibodies composed of LRRC15 antigen-binding domain and CD137 antigen-binding domain, as shown in a-d of Figure 3A.

A bispecific antibody of structure a in Figure 3A is named 15BBL-L06, and its structure is shown in Figure 3C.

A bispecific antibody of structure b is named 15BBTL-L06, and its structure is shown in Figure 3D.

A bispecific antibody of structure c is named 15BB-L06, and its structure is shown in Figure 3B. Another bispecific antibody of structure c is named 15BBe-L06, which differs from 15BB-L06 in that the anti-CD137 scFv uses the VH and VL of CD137e.

A bispecific antibody of structure d is named BB15-L06, and its structure is shown in Figure 3E. Another bispecific antibody of structure d is named BBe15-L06, which differs from BB15-L06 in that the anti-CD137 IgG uses the VH and VL of CD137e.

Exemplary molecules prepared in this example are shown in the following table:

| Molecule Name | Chain 1 (SEQ ID NO:) | Chain 2 (SEQ ID NO:) | Chain 3 (SEQ ID NO:) |
|---|---|---|---|
| 315-L06 | 61 | 80 | 83 |
| 153-L06 | 62 | 58 | - |
| 153-L07 | 63 | 58 | - |
| 153-L06C | 64 | 58 | - |
| 315-L0608 | 62 | 81 | 84 |
| 315-L0806 | 65 | 80 | 83 |
| 315-L08C06 | 66 | 80 | 83 |
| 315-L06C08 | 64 | 81 | 84 |
| 28315-L06 | 67 | 80 | 83 |
| 1540L-L06 | 68 | - | 83 |
| 1540TL-L06 | 69 | 82 | 83 |
| 1540-L06 | 70 | - | 83 |
| 4015-L06 | 71 | - | 85 |
| 40D15-L06C | 72 | - | 86 |
| 40D15-L06CS | 73 | - | 86 |
| 15BBL-L06 | 74 | - | 83 |
| 15BBTL-L06 | 75 | 82 | 83 |
| 15BBe-L06 | 76 | - | 83 |
| 15BB-L06 | 77 | - | 83 |
| BBel5-L06 | 78 | - | 87 |
| BB15-L06 | 79 | - | 88 |

### Example 3

### Binding verification of bispecific antibody molecules targeting LRRC15 and CD3 with target molecules

The binding of bispecific antibody molecules targeting LRRC15 and CD3 (153-L06C, with a structure shown in Figure 1B; 315-L06C08, with a structure shown in Figure 1C), a control sample BM bispecific molecule (its sequences refer to WO2021022304A2) with CHO cells expressing human LRRC15 and CHO cells expressing cynomolgus LRRC15 were detected, and the method was as described in Example 1.

The results showed that 315-L06C08 exhibited the lowest EC50 value for the CHO cells expressing human LRRC15, indicating the strongest binding affinity. Conversely, 153-L06C demonstrated the highest binding platform period, meaning it could bind more antibody molecules at saturation dose. (Figure 5A).

For CHO cells expressing cynomolgus LRRC15, the binding trend of each molecule was similar to that of CHO cells expressing human LRRC15 (Figure 5B).

### Example 4

### Characterization of bispecific antibody molecules targeting LRRC15 and CD3

4.1 T cell activation assay of bispecific antibody molecules targeting LRRC15 and CD3

The T cell activation ability of bispecific antibody molecules targeting LRRC15 and CD3 (153-L06C and 315-L06C08) was measured, with the above BM molecule as a control sample. The specific method is as follows:
CHO cells expressing LRRC15 (wild-type CHO cells not expressing LRRC15 were used as a control) were washed with PBMC buffer (RPMI-1640+10%FBS+1% Penstrep) and resuspended with gradient diluted antibodies to be tested at 2E6 cells/ml. The prepared PBMC cells were added to the resuspension to make the E/T cell ratio of 10:1, and incubated at 37°C for 48h, then the cells were washed twice with FACS buffer and co-incubated with anti-CD3 and anti-CD25 secondary antibodies on ice for 30min. After washing the resuspended cells with FACS buffer again, analysis was conducted by flow cytometry. Based on the measured mean fluorescence intensity, the half maximal activation concentration (EC50) of each antibody was calculated.
CD25 is an important marker of T cell activation. The results showed that the three bispecific molecules induced T cell activation only in the presence of target cells. None exhibited activation effects on T cells in the presence of cells lacking the target protein expression. Among them, BM had the lowest EC50 for T cell activation, but the lowest maximum proportion of activated cells. 153-L06C required the highest dose for activation but also achieved the largest maximum proportion of activated cells. 315-L06C08 fell in the middle of the other two molecules(Figure 6).

### 4.2 IFN-y release assay of bispecific antibody molecules targeting LRRC15 and CD3

The ability of bispecific antibody molecules targeting LRRC15 and CD3 (153-L06C and 315-L06C08) to stimulate PBMCs to release IFN-y was measured, with the above BM molecule as a control sample. The specific method is as follows:
CHO cells expressing LRRC15 (wild-type CHO cells not expressing LRRC15 were used as a control) were washed with PBMC buffer (RPMI-1640+10%FBS+1% Penstrep) and resuspended with gradient diluted antibodies to be tested at 2E6 cells/ml. The prepared PBMC cells were added to the resuspension to make the E/T cell ratio of 10:1, and incubated at 37°C for 48h. The supernatant was taken to test the IFN-y content using IFN-y ELISA kit.

The results showed that the three bispecific molecules could stimulate PBMCs to release IFN-y only in the presence of target cells. None induced any significant IFN-y release in the absence of target cells. Among them, BM exhibited the weakest stimulatory effect. 153-L06C achieved the highest peak IFN-y release, but there was a large gap compared to the EC50 value of 315-L06C08 for inducing IFN-y release (Figure 7).

### 4.3 T cell proliferation assay stimulated by bispecific antibody molecules targeting LRRC15 and CD3

The ability of bispecific antibody molecules targeting LRRC15 and CD3 (153-L06C and 315-L06C08) to stimulate T cell proliferation was measured, with the above BM molecule as a control sample. The specific method is as follows:
T cells were isolated from PBMCs using a Pan T Cell Isolation Kit (cat# 130-096-535, Miltenyi) and labeled with CFSE. 50 µl of labeled T cells (containing 5E4 cells) were mixed with 100 µl of CHO cells expressing LRRC15 (containing 1E4 cells) (wild-type CHO cells not expressing LRRC15 were used as a control) in each well of a 96-well plate, and resuspended with 50 µl of gradient diluted antibodies to be tested, then subjected to incubation at 37°C for 5 days. Then the cells were washed twice with FACS buffer and analyzed by flow cytometry. Based on the proliferation of T cells under different conditions, the half-maximal proliferation-promoting concentration (EC50) of each antibody was determined according to the corresponding CFSE staining signal value.

The results showed that 153-L06C and 315-L06C08 could promote cell proliferation only in the presence of target cells. None of the three bispecific molecules significantly promoted proliferation in the presence of cells lacking LRRC15 expression. Among them, BM had almost no effect on T cell proliferation. 153-L06C achieved the highest maximum proportion of proliferating T cells, while it exhibited a large gap compared to the EC50 value of 315-L06C08 for promoting proliferation (Figure 8).

### 4.4 Cytotoxicity assay of PBMCs on target cells stimulated by bispecific antibody molecules targeting LRRC15 and CD3

The ability of bispecific antibody molecules targeting LRRC15 and CD3 (153-L06C and 315-L06C08) to stimulate PBMCs to kill target cells was measured, with the above BM molecule as a control sample. The specific method is as follows:
CHO cells expressing LRRC15 were washed with PBMC buffer (RPMI-1640+10%FBS+1% Penstrep) and resuspended with gradient diluted antibodies to be tested at 2E6 cells/ml. The prepared PBMC cells were added to the resuspension to make the E/T cell ratio of 10:1, then subjected to incubation at 37°C for 48h. CytoTox-GloTM cytotoxicity assay reagent was added to the incubated 96-well plate. Based on the measured chemiluminescence intensity, the killing effect of each antibody on target cells was determined.

The results demonstrated that all three bispecific molecules could stimulate PBMCs to kill target cells, but only in their presence. None exhibited significant killing activity against cells lacking LRRC15 expression. BM displayed the weakest killing effect and exhibited a significant hook effect. 153-L06C achieved the highest maximum proportion of killing, while it showed a large gap compared to the EC50 value of 315-L06C08 for inducing killing (Figure 9).

### 4.5 Cytokine release assay of PBMCs stimulated by bispecific antibody molecules targeting LRRC15 and CD3

The ability of bispecific antibody molecules targeting LRRC15 and CD3 (153-L06C and 315-L06C08, with structures shown in Figures 1B and 1C, respectively) to stimulate PBMC to release cytokines was measured, with the above BM molecule as a control sample, and CD3 antibody (OKT3) as a positive control. The specific method is as follows:
PBMC cells from 4 different donors (D1, D2, D3, D4) were thawed and diluted to 10E7 cells/ml with PBMC buffer (RPMI-1640+10%FBS+1% Penstrep) in a 96-well plate, and the plate was placed in a 37°C incubator for 48 hours. The antibody to be tested was diluted to 10nM with PBMC buffer and added to the 96-well plate containing PBMC cells, then subjected to incubation at 37°C for 24 hours. The BD^{™} Cytometric Bead Array (CBA) kit was used to capture and detect the cytokine content in the culture supernatant, which could show the cytokine release of different donor PBMCs stimulated by each antibody.

The results showed that the three bispecific molecules had no significant promoting effect on the release of 6 cytokines, while the CD3 antibody (OKT3) as a positive control had different degrees of cytokine release. Untreated was the group without addition of antibody, and as a negative control, there was no significant cytokine release (Figure 10).

### Example 5

### Binding verification of bispecific antibody molecules targeting LRRC15 and CD137/CD40 with target molecules

The binding of bispecific antibody molecules targeting LRRC15 and CD137 (15BB-L06, with a structure shown in Figure 3B) to 4-1BB protein, and the binding of bispecific antibody molecules targeting LRRC15 and CD40 (1540-L06, with a structure shown in Figure 2B) to CD40 protein were detected by ELISA method. The specific method is as follows:
A 96-well plate was coated with 4-1BB or CD40 antigen proteins overnight at 4°C, then the 96-well plate was washed three times with 0.05% PBST solution, and added with 200 µl of 1% BSA to each well for blocking at room temperature for 1h. The plate was washed three times with 0.05% PBST again. Gradient diluted antibody molecules (15BB-L06 or 1540-L06) were resuspended with 1% BSA and added to the 96-well plate in duplicate wells at 100 µl per well. The plate was washed three times with 0.05% PBST, then added with anti-hIgG-HRP secondary antibody and incubated at room temperature for 0.5h. The plate was washed three times with 0.05% PBST, then added with TMB reagent for color development and the value at 450nm in an enzyme reader was read. Based on the measured mean OD450 value, the half maximal binding concentration (EC50) of each antibody was determined.

The results showed that 15BB-L06 could bind to 4-1BB, and 1540-L06 could bind to CD40 (Figure 11). Moreover, the 4-1BB antibody or CD40 antibody part in the bispecific antibody molecule is in the form of scFv, which will not lead to over-activation after the antibody binds to the antigen compared with its IgG form, thereby reducing the risk of the bispecific antibody molecule during administration.

### Example 6

### Characterization of bispecific antibody molecules targeting LRRC15 and CD137/CD40

The ability of bispecific antibody targeting LRRC15 and CD137 (15BB-L06) to stimulate PBMCs to release IL-2, and the ability of bispecific antibody targeting LRRC15 and CD40 (1540-L06) to stimulate DCs to release IL-12p40 were measured.

10 µg/ml of CD3 antibody (OKT3, eBioscience) was added to each well of a 96-well plate, at 100 µl per well, and incubated at 37°C for 2h. CHO cells expressing LRRC15 (wild-type CHO cells not expressing LRRC15 were used as a control) were washed with PBMC buffer (RPMI-1640+10%FBS+1% Penstrep) and resuspended with gradient diluted antibodies to be tested at 2E6 cells/ml. The prepared PBMC cells were added to the resuspension to make the E/T cell ratio of 10:1, then subjected to incubation at 37°C for 48h. The supernatant was taken and the IL-2 content was detected using the IL-2 ELISA kit.

The results showed that in the presence of target cells, 15BB-L06 stimulated higher IL-2 release than Urelumab (4-1BB monospecific antibody), but in the presence of cells not expressing the target protein, IL-2 release was similar to that of control IgG, demonstrating the effectiveness of the antibody molecule's target specificity. In contrast, the control antibody Urelumab did not have this specificity (Figure 12).

Monocytes were isolated from PBMCs using a monocyte separation kit (cat#130-096-537, Miltenyi). GM-CSF and IL-4 were added at 20ng/ml to induce the differentiation of monocytes into DC cells, and DC cells were harvested after 7 days. CHO cells expressing LRRC15 (wild-type CHO cells not expressing LRRC15 were used as a control) were washed with PBMC buffer (RPMI-1640+10%FBS+1% Penstrep) and resuspended with gradient diluted antibodies to be tested at 2E6 cells/ml. The prepared DC cells were added to the resuspension to make the E/T cell ratio of 3:1, and subjected to incubation at 37°C for 24h. The supernatant was taken and the IL-12p40 content was detected using the IL-12p40 ELISA kit.

The results showed that although 1540-L06 stimulated slightly lower IL-12p40 release than Selicrelumab (CD40 monospecific antibody) in the presence of target cells, IL-12p40 release was significantly reduced in the presence of cells not expressing the target protein, demonstrating the effectiveness of the antibody molecule's target specificity. In contrast, the control antibody Selicrelumab did not have this specificity (Figure 13).

In addition, the ability of the above bispecific molecules to stimulate PBMCs to release cytokines (TNF-α and IL-6) was measured, with CD3 antibody (OKT3) as a positive control. The specific method is as follows:
PBMC cells from 5 different donors were thawed and diluted to 10E7 cells/ml with PBMC buffer (RPMI-1640+10%FBS+1% Penstrep) in a 96-well plate, and the plate was placed in a 37°C incubator for 48h. The antibody to be tested was diluted to 10nM with PBMC buffer and added to the 96-well plate containing PBMC cells, then subjected to incubation at 37°C for 24 hours. TNF-α and IL-6 ELISA kits were used to capture and detect the cytokine content in the culture supernatant, respectively, which could show the cytokine release of different donor PBMCs stimulated by each antibody.

The results showed that the two bispecific molecules 1540-L06 and 15BB-L06 had no significant promoting effect on the release of the two cytokines TNF-α and IL-6 (Figure 14).

### Example 7

### In vivo efficacy study of bispecific antibody molecules targeting LRRC15

### 7.1 NCI-H1650 model

NOG mice were inoculated with NCI-H1650 cells (1×10⁷ cells/mouse), and PBMCs were inoculated 15 days later. When the average tumor volume grew to about 200mm³, the mice were grouped and administered (the control group was administered with PBS, and the experimental groups were administered with 153-L06C and 315-L06C08, respectively). The administration was conducted by intravenous injection, twice a week, for 7 consecutive weeks. During this period, the tumor volume was measured (Figure 15A) and the mice were observed for any abnormalities.

The results showed that at equimolar doses, 153-L06C (0.2 mg/kg) and 315-L06C08 (0.288 mg/kg) showed similar antitumor effects, with TGIs of 37% and 39%, respectively. The animals tolerated the drug well during the administration period. At the end of the experiment, the tumors were collected and weighed. The tumor weight results showed more significantly the antitumor effects of 153-L06C and 315-L06C08, with tumor weights of 55% and 57% of that of the control group, respectively (Figure 15B).

### 7.2 MC38 model

Humanized hCD3e/h4-1BB double-gene knock-in C57BL/6 mice were inoculated with MC38-huLRRC15 cells (2×10⁶ cells/mouse), and when the average tumor volume grew to about 120 mm³, the mice were grouped and administered (the control group was administered with saline, and the experimental groups were administered with 153-L06C and 15BB-L06, respectively). The administration was conducted by intravenous injection, twice a week, for 4 consecutive weeks.

The results showed that 153-L06C (1 mg/kg) and 15BB-L06 (1 mg/kg) showed similar antitumor effects, with TGIs of 24% and 43%, respectively (Figure 15C).

Humanized hCD40 gene knock-in C57BL/6 mice were inoculated with MC38-huLRRC15 cells (2×10⁶ cells/mouse), and when the average tumor volume grew to about 120 mm³, the mice were grouped and administered (the control group was administered with saline, and the experimental group was administered with 1540-L06). The administration was conducted by intravenous injection, twice a week (the first two doses were 1 mg/kg, and the third dose was 6 mg/kg), for 3 consecutive weeks. After the dose was adjusted to 6 mg/kg, 1540-L06 showed a significant antitumor effect, with a TGI of 59% (Figure 15D).

### Comparison Example 1

### Comparative study of multi-specific T cell engager molecules with different structures

The ability of the bispecific antibody molecules targeting LRRC15 and CD3 (153-L06 and 315-L06) prepared in Example 2 to stimulate PBMCs to kill target cells was measured. The specific method refers to Example 4.4.

The results showed that the killing effect of 153-L06 was significantly better than that of 315-L06, indicating that the molecule with the structure of 153-L06 has a better ability to stimulate target cell killing (Figure 16).

The ability of the bispecific antibody molecules targeting LRRC15 and CD3 (153-L06C, 315-L06C08, 315-L08C06) and the trispecific antibody molecule targeting LRRC15, CD3 and CD28 (28315-L06) prepared in Example 2 to stimulate PBMCs to kill target cells was measured. The specific method refers to Example 4.4.

The results showed that the killing effect of 315-L06C08 was significantly better than that of other molecules, indicating that the molecule with the structure of 315-L06C08 has a better ability to stimulate target cell killing (Figure 17).

All of the references mentioned in this invention are incorporated herein by reference as if each individual reference were specifically and individually incorporated by reference. It should also be understood that after reading the foregoing disclosure of the invention, those skilled in the art can make various changes or modifications to the invention, and these equivalent forms are also within the scope of the claims appended to this application.

## Claims

1. A multi-specific T cell engager, which comprises:
A first targeting domain comprising one or more LRRC15 antigen binding domains;
A second targeting domain, wherein the second targeting domain binds to a target protein selected from the group consisting of: CD3, CD28, CD40, and CD137; and/or
An optional third targeting domain, wherein the third targeting domain binds to a target protein selected from the group consisting of: CD3, CD28, CD40, and CD137.

2. The multi-specific T cell engager of claim 1, wherein the targeting domain is in the form of a single-domain antibody (sdAb), a fragment variable (Fv) heterodimer, a single-chain Fv (scFv), a Fab fragment, a TriFab, or a combination thereof.

3. The multi-specific T cell engager of claim 1, wherein the LRRC15 antigen binding domain comprises a heavy chain variable region having at least 80% sequence identity to the amino acid sequence shown in SEQ ID NO: 1, 2, 3, 4, or 53, and a light chain variable region having at least 80% sequence identity to the amino acid sequence shown in SEQ ID NO: 5, 6, 7, 8, or 54.

4. The multi-specific T cell engager of claim 3, wherein the LRRC15 antigen binding domain comprises a heavy chain variable region as shown in SEQ ID NO: 1, and a light chain variable region as shown in SEQ ID NO: 5.

5. The multi-specific T cell engager of claim 3, wherein the LRRC15 antigen binding domain comprises a heavy chain variable region as shown in SEQ ID NO: 53, and a light chain variable region as shown in SEQ ID NO: 54.

6. The multi-specific T cell engager of claim 1, wherein the second targeting domain is selected from the group consisting of: a CD3 antigen binding domain, a CD28 antigen binding domain, a CD40 antigen binding domain, a CD 137 antigen binding domain, a CD40L sequence, and a CD137L sequence.

7. The multi-specific T cell engager of claim 1, wherein the third targeting domain is selected from the group consisting of: a CD3 antigen binding domain, a CD28 antigen binding domain, a CD40 antigen binding domain, a CD 137 antigen binding domain, a CD40L sequence, and a CD137L sequence.

8. An anti-LRRC15 antibody or antigen-binding fragment thereof, which comprises the following three heavy chain variable region CDRs:
HCDR1 having an amino acid sequence as shown in SEQ ID NO: 32, 35, 38, or 41;
HCDR2 having an amino acid sequence as shown in SEQ ID NO: 33, 36, 39, or 42; and
HCDR3 having an amino acid sequence as shown in SEQ ID NO: 34, 37, 40, or 43;
and the following three light chain variable region CDRs:
LCDR1 having an amino acid sequence as shown in SEQ ID NO: 44 or 49;
LCDR2 having an amino acid sequence as shown in SEQ ID NO: 45, 47, or 50; and
LCDR3 having an amino acid sequence as shown in SEQ ID NO: 46, 48, 51, or 52.

9. Use of the multi-specific T cell engager of claim 1 for the preparation of a medicament for the treatment of cancer/tumor.

10. The use of claim 9, wherein the cancer/tumor is a cancer/tumor that highly expresses LRRC15.

11. An immunoconjugate comprising:
(i) the multi-specific T cell engager of claim 1, or the antibody or antigen-binding fragment thereof of claim 8; and
(ii) a coupling moiety selected from the group consisting of: a detectable label, a drug, a toxin, a cytokine, a radioisotope, and an enzyme.

12. A pharmaceutical composition comprising: (a) the multi-specific T cell engager of claim 1, or the antibody or antigen-binding fragment thereof of claim 8; and (b) a pharmaceutically acceptable carrier.
